# EUROPEAN PATENT APPLICATION

(11) **EP 0 978 280 A1**
(43) Date of publication of application: **09.02.2000**
(21) Application number: 98914114.8
(22) Date of filing: 21.04.1998
(51) Int. Cl.: A61K 31/435, A61K 31/44, A61K 31/495, A61K 31/505, C07D 453/06, C07D 471/08, C07D 401/04

(54) **2-AZABICYCLO COMPOUNDS**

(30) Priority: 26.04.1997 JP 12328097
(71) Applicant: Sumitomo Pharmaceuticals Company, Limited, Osaka 541-8510 (JP)
(72) Inventor: NISHIHARA, Toshio, Sumitomo Pharmaceuticals, Nishinomiya-shi Hyogo 662-0831 (JP); TOYODA, Tomohiro, Sumitomo Pharmaceuticals, Nishinomiya-shi Hyogo 662-0831 (JP)
(74) Representative: Cresswell, Thomas Anthony
(86) International application number: JP9801817
(87) International publication number: WO9848801

(57) **Abstract**

A 2-azabicyclo compound of the formula: 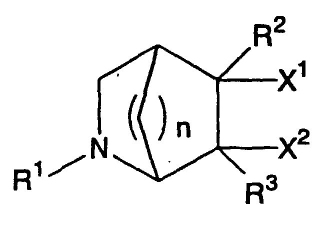
wherein R¹ is hydrogen or alkyl;
one of R² and R³ is optionally substituted nitrogen-containing heteroaryl; the other of R² and R³ is hydrogen, alkyl, alkenyl, alkynyl or aralkyl:
n is 1, 2 or 3;
one of X¹ and X² is hydrogen, and the other of X¹ and X² is hydrogen, hydroxy, halogen, alkoxy, alkenyloxy or alkynyloxy, or X¹ and X² are taken together with the solid line to form double bond;
or a pharmaceutically acceptable salt thereof is a nicotinic acetylcholine receptor agonist and is useful as a medicament for treating a neurodegenerative disease such as Alzheimer's disease, Parkinson's disease and the like, and as an analgesic.

## Description

### Technical Field

The present invention relates to a nicotinic acetylcholine receptor agonist useful as a medicament for treating a neurodegenerative disease such as Alzheimer's disease, Parkinson's disease and the like, and as an analgesic.

### Prior Art

The finding of marked hypofunction of acetyl-cholinergic neurons in the postmortem brain of patients with Alzheimer's disease gave impetus to explorations for the clinical application of acetylcholinesterase (AChE) inhibitors and muscarinic ACh receptor agonists, which are considered to stimulate the cholinergic nervous system, as drugs for the therapy of Alzheimer's disease.

Meanwhile, the relationship of changes in nicotinic ACh receptors to central neurodegenerative diseases such as Alzheimer's disease, Parkinson's disease, etc. is the current focus of attention. For example, it is reported that, in the brains of patients with Alzheimer's disease or Parkinson's disease, the number of nicotinic ACh receptors has been considerably decreased as compared with the number of muscarinic ACh receptors [J. Neurochem., 46, 288-293 (1986); Neurol., 38, 720 (1988)]. Furthermore, concerning the pharmacologic action of nicotine as a stimulant of nicotinic ACh receptors, it is reported that nicotine improves maze learning in rats with lesion of the Meynert's basal ganglia [Brain Res., 438, 83-94 (1988)], improves space cognitive learning in rats with lesion of the medial septum [Brain Res., 572, 281-285 (1992)], improves sample-matching behaviors in the aged monkeys [Neurobiol. Aging, 12, 233-238 (1991)], and inhibits neuronal degeneration in the rat lesion model of the nigral dopaminergic neurons [Prog. Brain Res., 79, 267 (1989)]. However, many of the nicotinic acetylcholine receptor agonists known today have adverse actions as well, thus inducing sedation, hypothermia and elevation of blood pressure, for instance.

Epibatidine, a natural product having 7-Azabicyclo[2. 2. 2]nonane skeleton (Mol. Pharmacol., 45, 563-569 (1994)), and 2-azabicyclo derivatives having a functional group such as pyridine, pyrimidine and the like at the C-3 position (WO 96/36637) are known as compounds having an affinity with nicotinic acetylcholine receptors.

JP 3-135978 (A) discloses that 6-(6-methoxypyridin-2-yl)-2-azabicyclo[2. 2. 2]octane and 6-chloro-6-(6-methoxypyridin-2-yl)-2-azabicyclo[2. 2. 2]octane stimulate muscarinic acetylcholine receptors.

JP 4-295477 (A) discloses that anti-6-(5-methoxypyrazin-2-yl)-2-azabicyclo[2. 2. 2]octane and anti-6-(6-methylpyrazin-2-yl)-2-azabicyclo[2. 2. 2]octane are useful for treating glaucoma.

### Description of the invention

The present invention is intended to provide a nicotinic acetylcholine receptor agonist useful as a medicament for treating a neurodegenerative disease such as Alzheimer's disease, Parkinson's disease and the like, and as an analgesic.

The inventors of the present invention have intensively carried out research, and found that 2-azabicyclo compounds are nicotinic acetylcholine receptor agonists useful as a medicament for treating a neurodegenerative disease such as Alzheimer's disease, Parkinson's disease and the like, and as an analgesic. Thus, the present invention has been accomplished.

The present, inventions are as follows:
[1] A nicotinic acetylcholine receptor agonist comprising a 2-azabicyclo compound of formula 1: 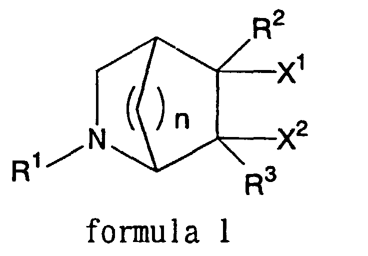
   wherein R¹ is hydrogen or alkyl;
   one of R² and R³ is optionally substituted nitrogen-containing heteroaryl; the other of R² and R³ is hydrogen, alkyl, alkenyl, alkynyl or aralkyl;
   n is 1, 2 or 3;
   one of X¹ and X² is hydrogen, and the other of X¹ and X² is hydrogen, hydroxy, halogen, alkoxy, alkenyloxy or alkynyloxy, or X¹ and X² are taken together with the solid line to form double bond;
   or a pharmaceutically acceptable salt thereof.
[2] A nicotinic acetylcholine receptor agonist according to [1] wherein one of R² and R³ is optionally substituted pyridyl, optionally substituted pyrazinyl, optionally substituted pyrimidinyl, optionally substituted pyridazinyl, optionally substituted quinolyl, optionally substituted isoxazolyl, optionally substituted isothiazolyl or optionally substituted pyridonyl.
[3] A nicotinic acetylcholine receptor agonist according to [2] wherein one of R² and R³ is 3-pyridyl, 5-pyrimidinyl, 2-pyrazinyl, 3-methyl-5-isoxazolyl, 5-ethynyl-3-pyridyl or 5-phenyl-3-pyridyl.
[4] A nicotinic acetylcholine receptor agonist according to any one of [1] to [3] wherein the other of R² and R³ is hydrogen.
[5] A nicotinic acetylcholine receptor agonist according to any one of [1] to [4] wherein n is 2.
[6] A nicotinic acetylcholine receptor agonist according to any one of [1] to [5] wherein both of X¹ and X² are hydrogen atoms or X¹ and X² are taken together with the solid line to form double bond.
[7] A nicotinic acetylchol me receptor agonist according to any one of [1] to [6] wherein R¹ is hydrogen.
[8] A method for stimulating nicotinic acetylcholine receptors comprising administrating to a patient in need thereof a 2-azabicyclo compound of the formula 1 or a pharmaceutically acceptable salt thereof.
[9] Use of a 2-azabicyclo compound of the formula 1 or a pharmaceutically acceptable salt thereof for the manufacture of a nicotinic acetylcholine receptor agonist.
[10] A 2-azabicyclo compound of formula 2: 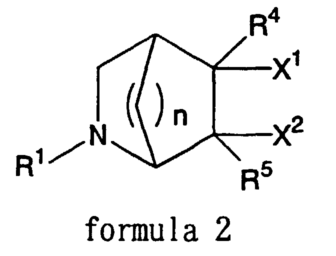
   wherein R¹, n, X¹ and X² are as defined in claim 1;
   one of R⁴ and R⁵ is optionally substituted 3-pyridyl, optionally substituted 5-pyrimidinyl, optionally substituted 2-pyrazinyl, optionally substituted 5-isoxazolyl or optionally substituted pyridonyl, provided that X¹ and X² are taken together with the solid line to form double bond incase that one of R⁴ and R⁵ is optionally substituted 2-pyrazinyl;
   the other of R⁴ and R⁵ is hydrogen, alkyl, alkenyl, alkynyl or aralkyl;
   or a pharmaceutically acceptable salt thereof.
[11] A 2-azabicyclo compound or a pharmaceutically acceptable salt thereof according to [10] wherein one of R⁴ and R⁵ is 3-pyridyl, 5-pyrimidinyl, 2-pyrazinyl, 3-methyl-5-isoxazolyl, 5-ethynyl-3-pyridyl or 5-phenyl-3-pyridyl.
[12] A 2-azabicyclo compound or a pharmaceutically acceptable salt thereof according to [10] or [11] wherein the other of R⁴ and R⁵ is hydrogen.
[13] A 2-azabicyclo compound or a pharmaceutically acceptable salt thereof according to any one of [10] to [12] wherein n is 2.
[14] A 2-azabicyclo compound or a pharmaceutically acceptable salt thereof according to any one of [10] to [13] wherein both of X¹ and X² are hydrogen atoms or X¹ and X² are taken together with the solid line to form double bond.
[15] A 2-azabicyclo compound or a pharmaceutically acceptable salt thereof according to any one of [10] to [14] wherein R¹ is hydrogen.
[16] A medicament comprising a 2-azabicyclo compound or a pharmaceutically acceptable salt thereof according to any one of [10] to [15].

Alkyl includes straight or branched C₁-C₆ alkyl. Typical examples are methyl, ethyl, propyl, 1-methylethyl, butyl, 1-methylpropyl, 2-methylpropyl, pentyl, 1-methylbutyl, 2-methylbutyl, 1-ethylpropyl, hexyl, 1-methylpentyl, 2-methylpentyl, 1-ethylbutyl and the like.

Alkenyl includes straight or branched C₂-C₆ alkenyl. Typical examples are vinyl, 1-propenyl, 2-propenyl, 3-pentenyl and the like.

Alkynyl includes straight or branched C₂-C₆ alkynyl. Typical examples are ethynyl, 1-propynyl, 2-propynyl, 3-butynyl and the like.

Alkoxy includes straight or branched C₁-C₆ alkoxy. Typical examples are methoxy, ethoxy, propoxy, 1-methylethoxy, butoxy, 1-methylpropoxy, 2-methylpropoxy, pentoxy, 1-methylbutoxy, 2-methylbutoxy, 1-ethylpropoxy, hexoxy, 1-methylpentoxy, 2-methylpentoxy, 1-ethylbutoxy and the like.

Alkenyloxy includes straight or branched C₂-C₆ alkenyloxy. Typical examples are vinyloxy, 1-propenyloxy, 2-propenyloxy, 3-pentenyloxy and the like.

Alkynyloxy includes straight or branched C₂-C₆ alkynyloxy. Typical examples are ethynyloxy, 1-propynyloxy, 2-propynyloxy, 3-butynyloxy and the like.

Alkanoyl includes straight or branched C₁-C₆ alkanoyl. Typical examples are formyl, acetyl, propanoyl, butanoyl and the like.

Aralkyl includes alkyl substituted by aryl. Typical examples are benzyl, 2-phenylethyl, 3-(2-naphthyl)propyl and the like.

Aryl includes C₆-C₁₀ aryl. Typical examples are phenyl, 1-naphthyl, 2-naphthyl and the like.

Nitrogen-containing heteroaryl includes, for example, 5- or 6-membered mono- or bi-cyclic heteroaryl containing nitrogen atom(s) and optionally oxygen atom(s) or sulfur atom(s). Typical examples are pyridyl, pyrazinyl, pyrimidinyl, pyridazinyl, quinolyl, isoxazolyl, isothiazolyl, pyridonyl and the like. Preferred examples are 2-pyridyl, 3-pyridyl, 4-pyridyl, 5-pyrimidinyl, 2-pyrazinyl, 3-pyridazinyl, 2-quinolyl, 3-quinolyl, 4-quinolyl, 5-isoxazolyl, 5-isothiazolyl, 2-pyridon-5-yl, 2-pyridon-3-yl and the like. More preferred examples are 3-pyridyl, 5-pyrimidinyl, 2-pyrazinyl, 5-isoxazolyl and the like.

The substituent of substituted nitrogen-containing heteroaryl, substituted pyridyl, substituted pyrazinyl, substituted pyrimidinyl, substituted pyridazinyl, substituted quinolyl, substituted isoxazolyl, substituted isothiazolyl and substituted pyridonyl includes, for example, alkyl, alkenyl, alkynyl, aralkyl, aryl, halogen, hydroxy, alkanoyl, alkoxy, amino, alkylamino, alkanoylamino, carboxyl, alkoxycarbonyl, cyano, alkyl substituted by halogen(s), carbamoyl, sulfamoyl and the like. One or more of these substituents may substitute independently.

Favorable examples of the substituents are alkyl, alkenyl, alkynyl, alkoxy, halogen, aryl and the like. Positions of the substitution are, for example, on any carbons which form the ring.

Halogen includes, for example, fluorine, chlorine, bromine, iodine and the like.

The pharmaceutically acceptable salt includes, for example, salt with inorganic acid such as hydrochloride, hydrobromide, sulfate, phosphate, nitrate and the like, and salt with organic acid such as acetate, propionate, succinate, lactate, malate, tartarate, citrate, maleate, fumarate, methanesulfonate, p-toluenesulfonate, ascorbate and the like.

The present invention includes the solvate such as a hydrate, a ethanolate and the like, of a 2-bicyclo compound of the formula 1 or a pharmaceutically acceptable salt thereof.

The 2-azabicyclo compound of the formula 1 can be produced, for example, by the following methods. Though it is illustrated below as a method of producing a 2-azabicyclo compound of the formula 1 wherein R² is hydrogen, alkyl, alkenyl, alkynyl or aralkyl, and R³ is optionally substituted heteroaryl, a 2-azabicyclo compound wherein R² and R³ have the opposite definitions can also be produced by the same method.

### Manufacture method 1

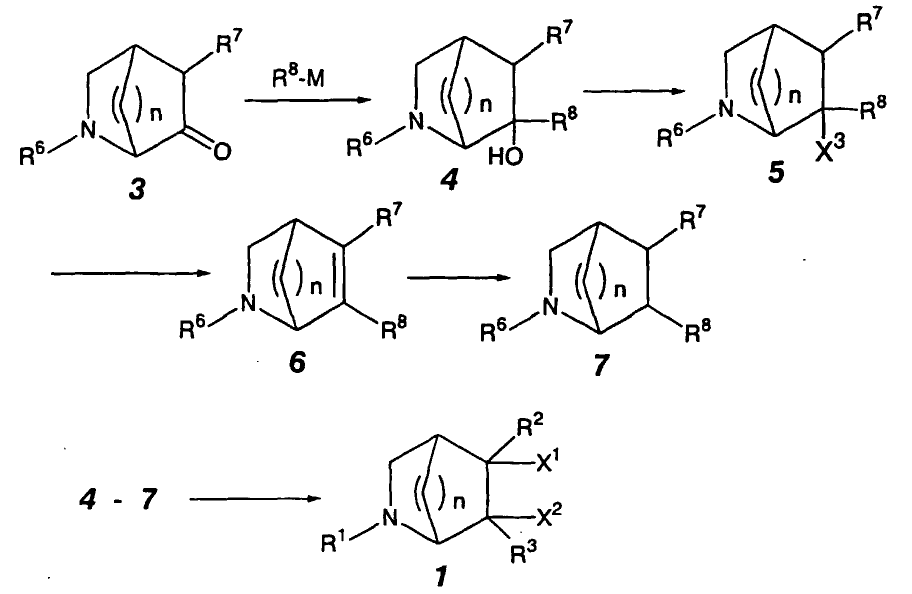

[wherein R¹, R², R³, X¹, X² and n are as defined above; R⁶ is alkyl or amino-protecting group; R⁷ is hydrogen, alkyl, alkenyl, alkynyl or aralkyl; R⁸ is optionally substituted heteroaryl; M is lithium atom, magnesium bromide or magnesium chloride; X³ is halogen atom.]
{1} A compound of the formula (4) can be produced by reacting a compound of formula: R⁸-M (R⁸ and M are as defined above) with a ketone of the formula (3), for example, at a temperature from -78 °C to room temperature. The reaction solvent includes ethers such as tetrahydrofuran (THF) and the like and hydrocarbons such as toluene, hexane and the like.
   A ketone of the formula (3) wherein R⁷ is hydrogen can be produced according to the method described in Synthetic Communications, 2, 211(1972) or J. Org. Chem., 53, 2259(1988). Other ketones of the formula (3) can be produced by introducing the substituent R⁷ to this compound according to conventional methods. The amino-protecting group includes known protecting group (for example "Protective Groups in Organic Synthesis", T.W.Greene, A Wiley-Interscience Publication(1981)). Typical examples are ethoxycarbonyl, benzyloxycarbonyl, tert-butoxycarbonyl and the like. These protecting groups may be introduced and removed by a conventional method.
   A compound of the formula: R⁸-M (R⁸ and M are as defined above) can be produced, for example, by reacting n-, sec- or tert-butyl lithium at a temperature from -100 to -78 °C, or by reacting magnesium at a temperature from 0 to 50 °C, with a compound of the formula: R⁸-X (R⁸ is as defined above; X is bromine atom or chlorine atom).
{2} A compound of the formula (5) can be produced, for example, by reacting a halogenation agent such as thionyl chloride and the like with a compound of the formula (4). Then the compound of the formula (5) can be treated with a base such as pyridine, DBU, sodium hydroxide and the like in a reaction solvent such as 1,2-dichloroethane and the like or without solvent at a temperature from room temperature to 150 °C to provide a olefin of the formula (6).
   A compound of the formula (6) can also be produced by reacting a suitable dehydration agent such as Burgess reagent (J. Org. Chem., 38, 26(1973)) with a compound of the formula (4) in a solvent such as toluene and the like at a temperature from room temperature to 70 °C.
{3} A compound of the formula (7) can be produced by reducing a olefin of the formula (6). The reducing method includes hydrogenation in the presence of a catalyst such as palladium/carbon and the like in a reaction solvent such as ethyl acetate, ethanol and the like at a temperature from room temperature to 100 °C and the like.
   A compound of the formula (7) can be also produced by reducing a compound of the formula (5). The reducing method includes hydrogenation in the presence of a catalyst, reduction with zinc (reduction with zinc dust in acetic acid at a temperature from room temperature to 150 °C) and the like.
{4} In case that R⁶ is an amino-protecting group in the compound of the formula (4), (5), (6) or (7) as produced above, the protecting group may be removed to produce a 2-azabicyclo compound of the formula 1. A method of removing the protecting group includes hydrolysis with a base such as sodium hydroxide and the like at a temperature from 100 to 200 °C, the method with iodotrimethylsilane and the like.
   Alkylation may be carried out to a compound of the formula (1) wherein R¹ is hydrogen. The alkylation condition includes the method of heating with formal in in the presence of formic acid, the method of reacting alkyl halide in the presence of a suitable base and the like.
{5} A compound (7) can be also produced by directly reducing the hydroxyl group in a compound of the formula (4). The reducing method includes the method of reacting ethyl chlorooxalate in the presence of a base such as 4-dimethylaminopyridine and the like, followed by reacting tributyltin hydride in the presence of a catalytic amount of 2,2'-azobisisobutyronitrile and the like.

### Manufacture method 2

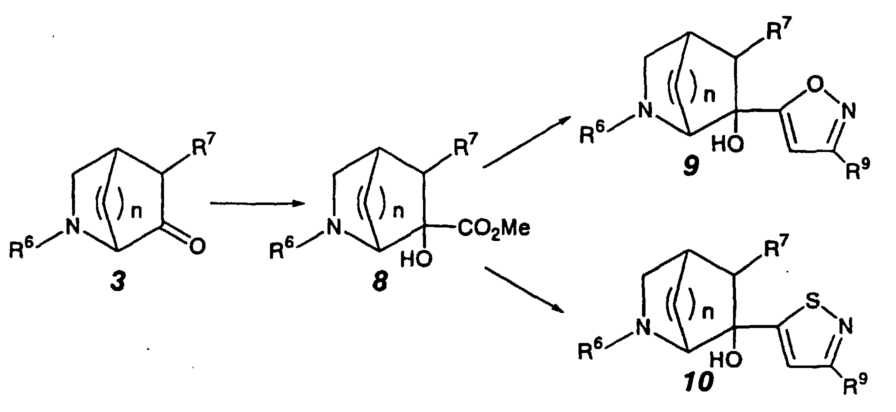

[wherein n, R⁶ and R⁷ are as defined above; R⁹ is hydrogen, alkyl, alkenyl, alkynyl or aralkyl.]
{1} An ester of the formula (8) can be produced, for example, by reacting potassium cyanide and the like with a ketone of the formula (3) in a solvent such as water, THF and the like at a temperature from 0 °C to room temperature to give the cyanohydrin, followed by reacting hydrogen chloride in methanol at a temperature from -78 to 0 °C.
{2} A compound of the formula (9) can be produced by carrying out an isoxazole ring formation reaction from a ester of the formula (8). The isoxazole ring formation reaction includes the method of reacting a compound of the formula (8) with the dianion formed by treating two molar equivalents of a base such as n-butyl lithium and the like to a compound of the formula: MeC(NOH)R⁹ (R⁹ is as defined above), followed by dehydration by treating with an acid and the like.
{3} A compound of the formula (10) can be produced by carrying out an isothiazole ring formation reaction from a ester of the formula (8). The isothiazole ring formation reaction includes a method described in J. Med. Chem., 35, 1550-1557(1992) or J. Med. Chem., 37, 4455-4463(1994) and the like.
{4} A compound of the formula (9) or (10) can be changed to the corresponding 2-azabicyclo compound in the same method as that of the production of a compound of the formula (5), (6), (7), (8) or (1) from compound (4).

In the above reaction, a functional group may be protected if needed. The protective group include known protective groups (for example, "Protective Groups in Organic Synthesis", T.W.Greene, A Wiley-Interscience Publication (1981)) and the like.

A 2-azabicyclo compound of the formula (1) produced according to the above method may be a mixture of diastereoisomers. In that case, each diasteroisomer can be separated and purified by a suitable purification method such as silica gel column chromatography and the like at the stage of a 2-azabicyclo compound of the formula (1) or an intermediate thereof.

A pharmaceutically acceptable salt of a 2-azabicyclo compound of the formula 1 can be formed by mixing the compound with a pharmaceutically acceptable acid such as hydrogen chloride, oxalic acid, methansulfonic acid and the like in a solvent such as water, methanol, ethanol, acetone and the like.

An optical isomer of a 2-azabicyclo compound of the formula (1) may be also obtained by a suitable optical resolution method, for example, by forming a salt with an optically active acid such as tartaric acid, followed by fractional recrystallization.

A 2-azabicyclo compound of the formula 1 or a pharmaceutically acceptable salt thereof maybe administered orally or parenterally (e.g. intravenously, subcutaneously, intramuscularly, topically, by suppository, percutaneously or intranasally). A composition for oral administration includes, for example, tablets, capsules, pills, granules, powders, solutions, suspensions and the like. A compositin for parenteral administration includes: for example, aqueous solutions for injection, oil solutions, ointments, creams, lotions, suppository, plasters and the like.

These compositions can be prepared by a conventional method, and may contain nonpoisonous inert carrier or excipient which is conventionally used in pharmaceutical field.

The dose varies depending on the patient's condition such as age, body weight and the like, the grade of the symptoms, administration route and the like. But a 2-azabicyclo compound of the formula 1 or a pharmaceutically acceptable salt thereof is usually administered to an adult in a dose of approximately 0.1-1000 mg, preferably 1-300 mg per day.

### Examples

The present invention will be described in detail below, referring to examples, which are not limitative of the present invention.

### Example 1

### Syn-6-(3-pyridyl)-2-azabicyclo[2. 2. 2]octane oxalate

### (1) 2-Ethoxycarbonyl-6-hydroxy-6-(3-pyridyl)-2-azabicyclo[2. 2. 2]octane

Under a nitrogen atmosphere, 2.0mL (3.2 mmol) of 1.6 N n-butyl lithium in hexane was added dropwise to a solution of 3-bromopyridine 530 mg (3.2 mmol) in 20 mL of THF at -78 °C, and stirred for 10 min. To the mixture was added at the same temperature a solution of 2-ethoxycarbonyl-2-azabicyclo[2. 2. 2]octan-6-one 530 mg(2. 7 mmol: Synthetic Communications, 2, 211(1972), J. Org. Chem., 53, 2259(1988)) in 3mL of THF and stirred for further 15 mm. After water was added to the mixture and warmed to room temperature, THF was evaporated in vacuo and the resultant aqueous layer was extracted with chloroform. The extracts were dried over anhydrous Na₂SO₄, concentrated in vacuo and the residue was purified by silica gel column chromatography (chloroform:methanol = 30:1 → 20:1) to give the titled compound 298 mg (yield 40%) as a brown oil.
¹H-NMR δ(CDCl₃, 270MHz) 0.80, 1.17 (total 3H, t, J=7Hz), 1.55-2.50 (6H, m), 3.30, 3.36 (total 1H, td, J=2, 11Hz), 3.72, 3.99 (total 2H, q, J=7Hz), 3.91, 4.14 (total 1H, t, J=3Hz), 7.20 (1H, dd, J=4.6, 8.3Hz), 7.65, 7.84 (total 1H, ddd, J=2, 3, 8Hz), 8.34-8.40 (1H, m), 8.59, 8.68 (total 1H, d, J=2Hz)

### (2) 2-Ethoxycarbonyl-6-(3-pyridyl)-2-azabicyclo[2. 2. 2]oct-5-ene

Two mL of 4N HCl/dioxane was added to a solution of 2-ethoxycarbonyl-6-hydroxy-6-(3-pyridyl)-2-azabicyclo[2. 2. 2.]octane 75 mg(0.27 mmol) in 1 mL of 1,4-dioxane and the mixture was concentrated in vacuo. To the residue was added thionyl chloride 3 mL at room temperature and heated under reflux for 7 hours. After thionyl chloride was distilled off in vacuo, water was added and neutralized with aq. 4 N NaOH solution. The mixture was extracted with chloroform, and extracts were dried over anhydrous Na₂SO₄, concentrated in vacuo and the residue was purified by silica gel column chromatography (chloroform:methanol = 50:1) to give the titled compound 38 mg(yield 54%) as a brown oil.
¹H-NMR δ(CDCl₃, 270MHz) 1.25, 1.31 (total 3H, t, J=7Hz), 1.50 (1H, br d, J=9Hz), 1.60-1.80 (1H, m), 2.02(1H, m), 2.89-3.00 (1H, m), 3.10 (1H, br t, J=10Hz), 3.36 (1H, dd, J=1.7, 9.9Hz), 4.05-4.25 (2H, m), 5.17, 5.27(total 1H, s), 6.67, 6.69 (total 1H, d, J=7Hz), 7.28 (1H, dd, J=4.7, 7.9Hz), 7.73, 7.83 (total 1H, br d, J=8Hz), 8.50, 8.73 (total 1H, br s)

### (3) 2-Ethoxycarbonyl-6-(3-pyridyl)-2-azabicyclo[2. 2. 2]octane

140 Milligram of 10 % palladium/carbon was added to a solution of 2-ethoxycarbonyl-6-(3-pyridyl)-2-azabicyclo[2. 2. 2]oct-5-ene 350 mg(1.36 mmol) in 3mL of ethyl acetate and the mixture was stirred under hydrogen atmosphere for 2 hours. The catalyst was filtered off through Celite® bed and the filtrate was concentrated in vacuo and the residue was purified by silica gel column chromatography(ethyl acetate) to give the titled compound 302 mg(yield 86%).
¹H-NMR δ(CDCl₃, 270MHz) 0.89, 1.23 (total 3H, t, J=7Hz), 1.60-2.30 (5H, m), 3.00-3.11 (1H, m), 3.38-3.62 (2H,m), 3.77-4.18 (3H, m), 7.22 (1H, dd, J=5.6, 6.9Hz), 7.48, 7.55 (total 1H, dd, J=2, 7.9Hz), 8.42-8.50 (2H, m)

### (4) Syn-6-(3-pyridyl)-2-azabicyclo[2. 2. 2]octane

A solution of 2-ethoxycarbonyl-6-(3-pyridyl)-2-azabicyclo[2. 2. 2]octane 300 mg(1.2 mmol) in methanol (4 mL) and aq. 4 N NaOH(3 mL) solution was stirred man autoclave at 150 to 200 °C for 9 hours. After methanol was evaporated in vacuo, the resultant aqueous layer was saturated with NaCl and was extracted with chloroform. The extracts were dried over anhydrous Na₂SO₄, concentrated in vacuo and the less polar isomer was purified from the residue by silica gel column chromatography (chloroform:methanol:triethylamine = 100:10:2 → 100:10:4) to give the free amine 112 mg as a pale yellow oil. This amine was dissolved in ethanol 1 mL. To this solution was added dropwise a solution of oxalic acid 20 mg in 3mL of ether. The precipitates formed were collected by filtration, washed with ether and dried to give the titled compound 130 mg as pale yellow powders.
¹H-NMR δ(D₂O, 270MHz) 1.77-1.87 (2H, m), 1.99-2.12 (3H, m), 2.15-2.25 (1H, m), 2.37-2.51 (1H, m), 3.23 (2H, br s), 3.64 (1H, dd, J=7.9, 11Hz), 3.86-3.92 (1H, m), 8.05 (1H, dd, J=5.9, 8.3Hz), 8.64 (1H, d, J=8.1Hz), 8.69 (1H, d, J=5.4Hz), 8.83 (1H, br s)

### Example 2

### 6-(3-Pyridyl)-2-azabicyclo[2. 2. 2]oct-5-ene

Two mL of aq. 4 N NaOH solution was added to a solution of 2-ethoxycarbonyl-6-(3-pyridyl)-2-azabicyclo[2. 2. 2]oct-5-ene 35 mg(0.14 mmol) obtained in Example 1(2) in 2 mL of methanol and the mixture was stirred in an autoclave at 140 to 200 °C for 2 hours. After methanol was evaporated in vacuo, the resultant aqueous layer was saturated with NaCl and extracted with chloroform. The extracts were dried over anhydrous Na₂SO₄ and concentrated in vacuo to give the titled compound 25 mg as a yellow oil.
¹H-NMR δ(CDCl₃, 270MHz) 1.32-1.55 (2H, m), 1.73 (1H, m), 2.03-2.15 (1H, m), 2.56 (1H, td, J=3, 9.9Hz), 2.75-2.82 (1H, m), 3.02 (1H, dd, J=1.3, 9.9Hz), 4.05 (1H, dd, J=3.0, 5.1Hz), 6.69 (1H, dd, J=2.0, 6.9Hz), 7.26 (1H, dd, J=4.8, 7.9Hz), 7.67 (1H, ddd, J=1.7, 2.3, 7.9Hz), 8.47 (1H, dd, J=1.7, 5.0Hz), 8.68 (1H, d, J=2.3Hz)

### Example 3

### Anti-6-(3-pyridyl)-2-azabicyclo[2. 2. 2]octane oxalate

The more polar isomer of free amine 21 mg was obtained as an oil from the crude product of Example 1(4) by purification with silica gel column chromatography (chloroform:methanol:triethylamine = 100:10:2 → 100:10:4). This amine was dissolved in ethanol 1 mL, and a solution of oxalic acid 20 mg in 3mL of ether was added dropwise. The precipitates formed were collected by filtration, washed with ether and dried to give the titled compound 33 mg as pale yellow powders.
¹H-NMR δ(D₂O, 270MHz) 1.65-2.10 (4H, m), 2.21 (1H, br s), 2.28-2.41 (1H, m), 3.34 (2H, br s), 3.65-3.80 (2H, m), 8.06 (1H, dd, J=5.6, 8.3Hz), 8.62 (1H, br d, J=8.2Hz), 8.70 (1H, br d, J=5.9Hz), 8.79 (1H, d, J=2Hz)

### Example 4

### Syn-5-(3-pyridyl)-2-azabicyclo[2. 2. 2]octane

### (1) 2-Ethoxycarbonyl-5-hydroxy-5-(3-pyridyl)-2-azabicyclo[2. 2. 2]octane

Under a nitrogen atmosphere, 14mL(23mmol) of 1.6 N n-butyl lithium in hexane was added dropwise to a solution of 3-bromopyridine 3.60 g (22.8 mmol) in 200mL of THF at -78 °C, and stirred for 10 min. To the mixture was added a solution of 2-ethoxycarbonyl-2-azabicyclo[2. 2. 2]octan-5-one(15.2 mmol: Synthetic Communications, 2, 211(1972)) 3.00 g in 30mL of THF at the same temperature, and stirred for further 1 hour. After water 20 mL was added and warmed to room temperature, THF was evaporated in vacuo and the resultant aqueous layer was extracted with chloroform. The extracts were dried over anhydrous Na₂SO₄, concentrated in vacuo and the residue was purified by silica gel column chromatography (ethyl acetate:methanol = 50:1) to give the titled compound 1.43 g(yield 34%) as a brown oil.
¹H-NMR δ(CDCl₃, 270MHz) 1.21, 1.28 (total 3H, t, J=7Hz), 1.50-3.35 (9H, m), 4.02-4.20 (2H, m), 4.26, 4.36 (total 1H, br s), 7.20-7.35 (1H, m), 7.72-7.90 (1H, m), 8.45-8.55 (1H, m), 8.75 (1H, br s)

### (2) 2-Ethoxycarbonyl-5-(3-pyridyl )-2-azabicyclo[2. 2. 2]oct-5-ene

Four mL of 4N HCl/dioxane was added to a solution of 2-ethoxycarbonyl-5-hydroxy-5-(3-pyridyl)-2-azabicyclo[2. 2. 2]octane 1.40 g(5.07 mmol) in 4 mL of 1,4-dioxane and concentrated in vacuo. To the residue was added thionyl chloride 4 mL and stirred at room temperature for 3.5 hours. After thionyl chloride was evaporated in vacuo, water was added and neutralized with 4 N NaOH aq. solution. The mixture was extracted with chloroform, and the extracts were dried over anhydrous Na₂SO₄, concentrated in vacuo and the residue was purified by silica gel column chromatography(ethyl acetate) to give the titled compound 950 mg(yield 73%) as a brown oil.
¹H-NMR δ(CDCl₃, 270MHz) 1.24, 1.29 (total 3H, t, J=7Hz), 1.40-2.20 (2H, m), 2.50-2.75, 3.10-3.22 (total 1H, m), 2.86, 3.28 (total 1H br s). 3.44 (1H, dd, J=2.0, 10.2Hz) , 4.00-4.40 (2H, m), 4.82, 4.94 (total 1H, br s), 6.70-6.82 (1H, m), 7.25-7.37 (1H, m), 7.69, 7.82-7.90 (total 1H, m), 8.52, 8.57 (total 1H, dd, J=2, 5Hz), 8.70, 8.83 (total 1H, br s)

### (3) 2-Ethoxycarbonyl-5-(3-pyridyl)-2-azabicyclo[2. 2. 2]octane

Forty mg of 10 % palladium/carbon was added to a solution of 2-ethoxycarbonyl-5-(3-pyridyl)-2-azabicyclo[2. 2. 2]oct-5-ene 100 mg(0.39 mmol) in 5 mL of ethyl acetate and the mixture was stirred under hydrogen atmosphere for 3.5 hours. The catalyst was filtered off through Celite® bed and the filtrate was concentrated in vacuo to give the titled compound 81 mg (yield 80%).
¹H-NMR δ(CDCl₃, 270MHz) 1.20-1.33 (3H, m), 1.45-2.45 (7H, m), 3.05-3.70 (3H, m), 4. 05-4.35 (3H, m), 7.28 (1H, br s), 7.55 (1H, br d, J=8.4Hz), 8.50 (2H, br s)

### (4) Syn-5-(3-pyridyl)-2-azabicyclo[2. 2. 2]octane oxalate

Two mL of aqueous 4N NaOH solution was added to a solution of 2-ethoxycarbonyl-5-(3-pyridyl)-2-azabicyclo[2. 2. 2]octane 400 mg(1.54 mmol) in 3 mL of methanol and the mixture was stirred in an autoclave at 150 to 180 °C for 5 hours. After methanol was evaporated in vacuo, the aqueous layer was saturated with NaCl and was extracted with chloroform. The extracts were dried over anhydrous Na₂SO₄ and concentrated in vacuo to give free amine (a mixture of diastereoisomers) 280 mg (yield 97%). A hundred mg of the free amine was purified by silica gel column chromatography(chloroform:methanol:triethylamine = 100:10:2 → 100:10:4) to give the less polar isomer of free amine 20 mg as an oil. This amine was dissolved in ethanol 1 mL. To this solution was added dropwise a solution of oxalic acid 20 mg in 3mL of ether. The precipitates formed were collected by filtration, washed with ether and dried to give the titled compound 23 mg as pale yellow powders.
¹H-NMR δ(D₂O, 270MHz) 1.80-2.23(5H, m), 2.28 (1H, br, s), 2.40-2.55 (1H, m), 3.17 (2H, br ABq, J=15Hz), 3.52 (1H, br t, J=9.4Hz), 3.69 (1H, br s), 8.05 (1H, dd, J=5.6, 8.3Hz), 8.59 (1H, d, J=7.9Hz), 8.68 (1H, d, J=5.6Hz), 8.76 (1H, s)

### Example 5

### 5-(3-Pyridyl)-2-azabicyclo[2. 2. 2]oct-5-ene

Three mL of aqueous 4N NaOH solution was added to a solution of 200mg(0.78 mmol) of 2-ethoxycarbonyl-5-(3-pyridyl)-2-azabicyclo[2. 2. 2]oct-5-ene obtained in Example 4(2) in 4 mL of methanol and the mixture was stirred in an autoclave at 130 to 150 °C for 3.5 hours. After methanol was evaporated in vacuo, the aqueous layer was saturated with NaCl and was extracted with chloroform. The extracts were dried over anhydrous Na₂SO₄ and concentrated in vacuo to give the titled compound 119 mg (yield 83%) as a yellow oil.
¹H-NMR δ(CDCl₃, 270MHz) 1.35-1.50 (2H, m), 1.75-2.10 (2H, m), 2.60 (1H, td, J=2.3, 10.2Hz), 3.07 (1H, dd, J=1.7, 10.2Hz), 3. 14 (1H, br s), 3.65-3.78 (2H, m), 6.81 (1H, dd, J=1.7, 5.9Hz), 7.26 (1H, dd, J=4.9, 7.9Hz), 7.70 (1H, br d, J=8.2Hz), 8.47 (1H, dd, J=1.3, 4.6Hz), 8.69 (1H, d, J=2.0Hz)

### Example 6

### Anti-5-(3-pyridyl)-2-azabicyclo[2. 2. 2]octane oxalate

Thirty mg of 10 % palladium/carbon was added to a solution of 5-(3-pyridyl)-2-azabicyclo[2. 2. 2]oct-5-ene 45 mg (0.24 mmol) obtained in Example 5 in 5 mL of methanol and the mixture was stirred under hydrogen atmosphere for 1 hour. After the catalyst was filtered off through Celite® bed, the filtrate was concentrated in vacuo and the residue was dissolved in ethanol 1 mL. This solution was added dropwise to a solution of 45 mg of oxalic acid in 2 mL of ether. The precipitates formed were collected by filtration, washed with ether and dried to give the titled compound 69 mg (yield 77%) as white powders.
¹H-NMR δ(D₂O, 270MHz) 1.50-2.15 (5H, m), 2.21 (1H, br, s), 2.45-2.63 (1H, m), 3.40 (2H, br ABq, J=13Hz), 3.48-3.58 (1H, m), 3.66 (1H, br s), 8.02 (1H, dd, J=5.9, 8.3Hz), 8.57 (1H, d, J=8.2Hz), 8.64 (1H, d, J=5.6Hz), 8.72 (1H, br s)

### Example 7

### 6-(2-Methoxy-5-pyridyl)-2-azabicyclo[2. 2. 2]oct-5-ene

### (1) 2-Ethoxycarbonyl-6-hydroxy-6-(2-methoxy-5-pyridyl)-2-azabicyclo[2. 2. 2]octane

Under a nitrogen atmosphere, 4.9 mL (7.7 mmol) of 1.6 N n-butyl lithium in hexane was added dropwise at -78 °C to a solution of 5-bromo-2-methoxypyridine 1.36 g (7.24 mmol: J. Am. Chem. Soc., 104, 4142 (1982)) in 50 mL of THF, and stirred for 50 min. To the mixture was added a solution of 2-ethoxycarbonyl-2-azabicyclo[2. 2. 2]octan-6-one 1.00 g (4.83 mmol) in 25 mL of THF dropwise over 45 min at the same temperature, and stirred for 15 min. After water was added to the mixture and warmed to room temperature, THF was evaporated in vacuo. The resultant aqueous layer was extracted with ethyl acetate. The extracts were washed with brine, dried over anhydrous MgSO₄, concentrated in vacuo and the residue was purified by silica gel column chromatography(hexane:ethyl acetate = 1:1) to give the titled compound 1.03 g (yield 70%) as a brown oil.
¹H-NMR δ(CDCl₃, 270MHz) 0.90, 1.18 (total 3H, t, J=6.9Hz), 1.50-2.50 (7H, m), 3.24-3.40 (2H, m), 3.75-4.23 (3H, m), 3.91 (3H, s), 6.70 (1H, dd, J=3.3, 8.6Hz), 7.63, 7.76 (total 1H, dd, J=3, 9Hz), 8.26 (1H, t, J=2.7Hz)

### (2) 2-Ethoxycarbonyl-6-(2-methoxy-5-pyridyl)-2-azabicyclo[2. 2. 2]oct-5-ene

To a solution of Burgess reagent 340mg (1.4 mmol: J. Org. Chem, 38, 26(1973)) in 2 mL of toluene was added dropwise a solution of 2-ethoxycarbonyl-6-hydroxy-6-(2-methoxy-5-pyridyl)-2-azabicyclo[2. 2. 2]octane 400 mg (1.3 mmol) in 1 mL of toluene and stirred at room temperature for 4. 5 hours. After water 5 mL was added, the mixture was extracted with ethyl acetate. The extracts were washed with brine, dried over anhydrous MgSO₄, concentrated in vacuo and the residue was purified by silica gel column chromatography (hexane:ethyl acetate = 2:1) to give the titled compound 345 mg (yield 92%) as a pale yellow oil.
¹H-NMR δ(CDCl₃, 270MHz) 1.20-1.35 (3H, m), 1.40-1.55 (2H, m), 1.65-1.80 (1H, m), 2.00-2.15 (1H, m), 2.85-2.95 (1H,m), 3.00-3.15 (1H, m), 3.34 (3H, dd, J=1.7, 9.9Hz), 3.94, 3.95 (total 3H, s), 4.05-4.25 (2H, m), 5.12, 5.22 (total 1H, s), 6.45-6.57 (1H, m), 6.73 (1H, d, J=8.6Hz), 7.60-7.80 (1H, m), 8.20-8.33 (1H, m)

### (3) 6-(2-Methoxy-5-pyridyl)-2-azabicyclo[2. 2. 2]oct-5-ene

Three mL of aqueous 4N NaOH solution was added to a solution of 2-ethoxycarbonyl-6-(2-methoxy-5-pyridyl)-2-azabicyclo[2. 2. 2]oct-5-ene 100 mg (0.35 mmol) in 5 mL of methanol and the mixture was stirred in an autoclave at 150 °C for 2 hours. After methanol was evaporated in vacuo, the resultant aqueous layer was extracted with chloroform. The extracts were dried over anhydrous Na₂SO₄, concentrated in vacuo and the residue was purified by silica gel column chromatography (chloroform:methanol:triethylamine = 100:10:2) to give the titled compound 48 mg (yield 64%) as a brown oil.
¹H-NMR δ(CDCl₃, 270MHz) 1.30-1.53 (2H, m), 1.66-1.78 (1H, m), 2.00-2.12 (1H, m), 2.54 (1H, td, J=3.0, 9.9Hz), 2.70-2.78 (1H, m), 3.00 (1H, dd, J= , 9.9Hz), 3.94 (3H, s), 3.99 (1H, dd, J=4.6, 7.6Hz) , 6.53 (1H, dd, J=2.0, 6.9Hz), 6.72 (1H, d, J=8.6Hz), 7.62 (1H, dd, J=2.6, 8.6Hz), 8.20 (1H, d, J=2.6Hz)

### Example 8

### 6-(2-Pyridon-5-yl)-2-azabicyclo[2. 2. 2]oct-5-ene hydrochloride

A solution of 6-(2-methoxy-5-pyridyl)-2-azabicyclo[2. 2. 2]oct-5-ene 37 mg (0.17 mmol) obtained in Example 7 in 2 mL of conc. hydrochloric acid was heated under reflux for 9.5 hours. The reaction mixture was concentrated in vacuo and isopropanol was added thereto. The precipitates formed were collected by filtration and dried to give the titled compound 36 mg (yield 89%) as white powders.
¹H-NMR δ(D₂O, 270MHz) 1.35-1.65 (2H, m), 1.70-1.82 (1H, m), 2.03-2.17 (1H, m), 2.79 (1H, br d, J=11Hz), 3.05 (1H, br d, J=4.9Hz), 3.20 (1H, br d, J=11Hz), 4.65 (1H, br s), 6.66 (1H, d, J=9.6Hz), 6.86 (1H, t, J=6.9Hz), 7.63 (1H, d, J=1.3Hz), 7.89 (1H, dd, J=2.6, 9.6Hz)

### Example 9

### 6-(3-Quinolyl)-2-azabicyclo[2. 2. 2]oct-5-ene

### (1) 2-Ethoxycarbonyl-6-hydroxy-6-(3-quinolyl)-2-azabicyclo[2. 2. 2]octane

Under a nitrogen atmosphere, 0.97 mL (1.5 mmol) of 1.6 N n-butyl lithium in hexane was added dropwise to a solution of 3-bromoquinoline 317 mg (1.5 mmol) in 10 mL of THF, at -78 °C, and stirred for 50 min. To the mixture was added dropwise a solution of 2-ethoxycarbonyl-2-azabicyclo[2. 2. 2]octan-6-one 200 mg (1.0 mmol) in 3 mL of THF, at the same temperature, and stirred for 25 min. After water was added to the mixture and warmed to room temperature, THF was evaporated in vacuo. The resultant aqueous layer was extracted with ethyl acetate. The extracts were washed with brine, dried over anhydrous MgSO₄, concentrated in vacuo and the residue was purified by silica gel column chromatography (ethyl acetate) to give the titled compound 64 mg (yield 20%) as a yellow oil.
¹H-NMR δ(CDCl₃, 270MHz) 0.53, 1.12 (total 3H, t, J=7Hz), 1.60-2.30 (5H, m), 2.38-2.55 (1H, m), 3.15-3.63 (2H, m), 3.95-4.36 (3H, m), 7.35-8.22 (5H, m), 8.89, 8.99 (total 1H, s)

### (2) 2-Ethoxycarbonyl-6-(3-quinolyl)-2-azabicyclo[2. 2. 2]oct-5-ene

Thionyl chloride 0.5 mL was added to a solution of 2-ethoxycarbonyl-6-hydroxy-6-(3-quinolyl)-2-azabicyclo[2. 2. 2]octane 64 mg (0.20 mmol) in 3 mL of pyridine and heated under reflux for 2 hours. After concentrated in vacuo, aqueous 1 N NaOH solution was added to the residue. The mixture was extracted with ethyl acetate. The extracts were dried over anhydrous Na₂SO₄, concentrated in vacuo and the residue was purified by silica gel column chromatography (hexane:ethyl acetate = 1:1) to give the titled compound 43 mg (yield 71%) as a yellow oil.
¹H-NMR δ(CDCl₃, 270MHz) 1.26, 1.36 (total 3H, t, J=7Hz ), 1.45-1.90 (3H, m), 2.10-2.30 (1H, m), 3.00 (1H, br s), 3. 10-3.23 (1H, m), 3.41 (1H, br d, J=10Hz), 4.05-4.30 (2H, m), 5.32, 5.45 (total 1H, br s), 6.86 (1H, d, J=6.6Hz), 7.50-7.91 (3H, m), 8.07-8.30 (2H, m), 9.08 (1H, br s)

### (3) 6-(3-Quinolyl)-2-azabicyclo[2. 2. 2]oct-5-ene

Three mL of aqueous 4N NaOH solution was added to a solution of 2-ethoxycarbonyl-6-(3-quinolyl)-2-azabicyclo[2. 2. 2]oct-5-ene 40 mg (0.13 mmol) in methanol 3 mL and the mixture was stirred in an autoclave at 150 °C for 3 hours. After methanol was evaporated in vacuo, the resultant aqueous layer was extracted with chloroform. The extracts were dried over anhydrous Na₂SO₄ and concentrated in vacuo to give the titled compound 29 mg (yield 95%) as a brown oil.
¹H-NMR δ(CDCl₃, 270MHz) 1.37-1.62 (2H, m), 1.72-1.85 (1H, m), 2.09-2.26 (1H, m), 2.63 (1H, br d, J=9.2Hz), 2.80-2.90 (1H, m), 3.07 (1H, br d, J=9.2Hz), 4.21 (1H, d, J=2.3Hz), 6.87 (1H, dd, J=2.0, 7.3Hz), 7.53 (1H, t, J=6.9Hz), 7.66 (1H, t, J=6.9Hz), 7.79 (1H, t, J=7.2Hz), 8.03 (1H, d, J=2.0Hz), 8.07 (1H, d, J=8.3Hz), 9.06 (1H, d, J=2.0Hz)

### Example 10

### 6-Hydroxy-6-(3-pyridyl)-2-azabicyclo[2. 2. 2]octane

One mL of aqueous 20% NaOH solution was added to a solution of 2-ethoxycarbonyl-6-hydroxy-6-(3-pyridyl)-2-azabicyclo[2. 2. 2]octane 87 mg (0.32mmol) obtained in Example 1(1) in 1 mL of methanol and the mixture was stirred in an autoclave at 200 °C for 2.5 hours. After methanol was evaporated in vacuo, the resultant aqueous layer was extracted with chloroform, dried over anhydrous Na₂SO₄. After the solvent was distilled off under reduced pressure, ether was added to the residue and the crystals formed were collected by filtration and dried to give the titled compound 42 mg (yield 68%) as pale yellow crystals.
¹H-NMR δ(CDCl₃, 270MHz) 1.70-2.00 (6H, m), 2.35 (1H, m), 2.48 (1H, td, J=2.3, 6.2Hz), 2.78 (1H, t, J=3.0Hz), 2.89 (1H, td, J=2.3, 10.6Hz), 3.00 (1H, td, J=2.0, 10.6Hz), 7. 29 (1H, dd, J=4.0, 8.6Hz), 7.93 (1H, ddd, J=1.7, 2.6, 8.9Hz), 8.51 (1H, dd, J=1.7, 4.6Hz), 8.84 (1H, d, J=2.3Hz)

### Example 11

### 2-Methyl-5-hydroxy-5-(3-pyridyl)-2-azabicyclo[2. 2. 2]octane

### (1) 5, 5-Ethylenedioxy-2-ethoxycarbonyl-2-azabicyclo[2. 2. 2]octane

To benzene 30 mL were added 2-ethoxycarbonyl-2-azabicyclo[2. 2. 2]octan-5-one 1.26 g (38.9 mmol), ethylene glycol 5 mL and p-toluenesulfonic acid 0.1 g, and the mixture was heated with azeotropic removal of water for 12 hours. The mixture was poured into 50 mL of aqueous 1 N NaOH solution. The organic layer was separated and the aqueous layer was extracted with toluene. The organic layers were combined, dried over anhydrous MgSO₄ and concentrated in vacuo to give the titled compound 1.42 g (yield 92%) as a colorless oil.

### (2) 5,5-Ethylenedioxy-2-methyl-2-azabicyclo[2. 2. 2]octane

Under a nitrogen atmosphere, to a suspension of lithium aluminum hydride 1.12 g (29.5 mmol) in 60 mL of THF was added dropwise a solution of 5,5-ethylenedioxy-2-ethoxycarbony-2-azabicyclo[2. 2. 2]octane 1.42 g (5.89 mmol) in 10 mL of THF, and the mixture was heated under reflux for 4 hours. Water was added to the mixture dropwise under cooling in an ice bath until generation of hydrogen ceased. After insoluble substances were filtered off through Celite® bed, the filtrate was concentrated in vacuo to give the titled compound 0.91 g (yield 84%) as a colorless oil.
¹H-NMR δ(CDCl₃, 270MHz) 1.40-1.97 (6H, m), 2.20-2.31 (1H, m), 2.34 (3H, s), 2.58-2.66 (2H, m), 2.91 (1H, td, J=3, 10Hz), 3.83 (4H, m)

### (3) 2-Methyl-2-azabicyclo[2. 2. 2]octan-5-one

To 20mL of 1 N hydrochloric acid was added 5,5-ethylenedioxy-2-methyl-2-azabicyclo[2. 2. 2]octane 0.85 g (4.6 mmol) and the mixture was heated under reflux for 7 hours. After neutralized with aq. 1 N NaOH solution under cooling in an ice bath and saturated with NaCl, the reaction mixture was extracted with ether. The extracts were dried over anhydrous Na₂SO₄ and concentrated in vacuo to give the titled compound 0.50 g (yield 77%) as a pale yellow oil.
¹H-NMR δ(CDCl₃, 270MHz) 1.52-1.64 (1H, m), 1.75-2.01 (2H, m), 2.12-2.44 (3H, m), 2.41 (3H, s), 2.49 (1H, td, J=3, 19Hz), 2.83 (1H, dd, J=2, 10Hz), 2.98 (1H, t, J=3Hz), 3.01 (1H, td, J=3,8Hz)

### (4) 2-Methyl-5-hydroxy-5-(3-pyridyl)-2-azabicyclo[2. 2. 2]octane

Under a nitrogen atmosphere, 3.4 mL (5.4 mmol) of 1.6 N n-butyl lithium in hexane was added dropwise to a solution of 3-bromopyridine 853 mg (5.4 mmol) in 8 mL of ether, at -78 °C, and stirred for 10 min. To the mixture was added dropwise a solution of 2-methyl-2-azabicyclo[2. 2. 2]octan-5-one 500 mg (3.6 mmol) in 5 mL of ether, at the same temperature, and stirred for further 20 min. After water was added to the mixture and waned to room temperature, the ether layer was separated and the aqueous layer was extracted with chloroform. The organic layers were combined, dried over anhydrous Na₂SO₄ and concentrated in vacuo. Ethyl acetate was added to the residue and the precipitates formed were collected by filtration to give the titled compound 1.67 g (yield 21%) as reddish brown powders.
¹H-NMR δ(CDCl₃, 270MHz) 1.56-1.73 (2H, m), 1.78 (1H, dd, J=3, 15Hz), 1.89 (1H, qu, J=2.7Hz), 1.98-2.35 (2H, m), 2.33 (3H, s), 2.46 (1H, dd, J=3, 11Hz), 2.51 (1H, dd, J=3, 11Hz), 2.74-2.83 (2H, m), 7.28 (1H, dd, J=5, 8Hz), 8.03 (1H, dt, J=2, 8Hz), 8.49 (1H, dd, J=2, 5Hz), 8.91 (1H, d J=2Hz)

### Example 12

### 2-Methyl-5-(3-pyridyl)-2-azabicyclo[2. 2. 2]oct-5-ene

One mL of 4 N HCl/dioxane was added to a solution of 2-methyl-5-hydroxy-5-(3-pyridyl)-2-azabicyclo[2. 2. 2]octane 80 mg (0.37 mmol) obtained in Example 11 in 1 mL of 1,4-dioxane and the mixture was concentrated in vacuo. Thionyl chloride 4 mL was added to the residue and the mixture was heated under reflux for 3.5 hours. After thionyl chloride was evaporated in vacuo, water was added to the residue and the mixture was neutralized with aqueous 1 N NaOH solution, and extracted with chloroform. The extracts were dried over anhydrous Na₂SO₄, concentrated in vacuo to give the titled compound 60 mg (yield 82%) as deliquescent brown powders.
¹H-NMR δ(CDCl₃, 270MHz) 1.25-1.48 (2H, m), 1.55-1.85 (1H, m), 1.95-2.15 (2H, m), 2.27 (3H, s), 3.06 (1H, d, J=2Hz), 3.20 (1H, dd, J=2, 10Hz), 3.42-3.50 (1H, m), 6.71 (1H, dd, J=2, 6Hz), 7.27 (1H, dd, J=5, 8Hz), 7.71 (1H, dt, J=2, 8Hz), 8.48 (1H, dd, J=2, 5Hz), 8.70 (1H, d, J=2Hz)

### Example 13

### 5-Benzyl-6-(3-pyridyl)-2-azabicyclo[2. 2. 2]oct-5-ene

### (1) 5-Benzyl-2-ethoxycarbonyl-2-azabicyclo[2. 2. 2]octan-6-one

Under a nitrogen atmosphere, to a solution of 2-ethoxycarbonyl-2-azabicyclo[2. 2. 2]octan-6-one 0.59 g (3 mmol) in 10 mL of THF was added dropwise 6 mL (3 mmol) of a solution of 0.5 M potassium hexamethyldisilazide in toluene at -78 °C, and stirred for 20 min. To the mixture was added benzyl bromide 0.53 mL (4.5 mmol) at the same temperature, stirred for further 30 min and warmed gradually to room temperature. The reaction mixture was poured into water, and extracted with ethyl acetate. The extracts were concentrated in vacuo and the residue was purified by silica gel column chromatography (hexane:ethyl acetate = 6:1) to give 5-benzyl-6-benzyloxy-2-ethoxycarbonyl-2-azabicyclo[2. 2. 2]oct-5-ene 0.25 g. This compound was dissolved in acetonitrile 2 mL and two drops of 1 N hydrochloric acid were added thereto. The mixture was stirred at room temperature for 15 min, concentrated in vacuo and the residue was purified by silica gel column chromatography (hexane:ethyl acetate = 5:1) to give the titled compound 0.17 g (yield 20%) as an oil.
¹H-NMR δ(CDCl₃, 270MHz) 1.25 (3H, m), 1.45-2.25 (5H, m), 2.56 (2H, m), 3.38, 3.77 (total 3H, m), 4.15 (2H, m), 4.29, 4.42 (total 1H, m), 7.16-7.38 (5H, m)

### (2) 2-Ethoxycarbonyl-5-benzyl-6-(3-pyridyl)-2-azabicyclo[2. 2. 2]oct-5-ene

Under a nitrogen atmosphere, to a solution of 3-bromopyridine 140 mg (0.90 mmol) in 5 mL of THF was added dropwise 0.56 mL (0.90 mmol) of 1.6 N n-butyl lithium in hexane at -78 °C, and stirred for 10 min. To the mixture was added a solution of 5-benzyl-2-ethoxycarbonyl-2-azabicyclo[2. 2. 2]octan-6-one 170 mg (0.59 mmol) in 3 mL of THF dropwise at the same temperature, and stirred for further 1 hour. The reaction mixture was poured into water, extracted with ethyl acetate. The extracts were washed with brine, dried over anhydrous MgSO₄ and concentrated in vacuo. The residue was dissolved in pyridine 3 mL and thionyl chloride 0.5 mL was added thereto and heated under reflux for 14 hours. The reaction mixture was concentrated in vacuo and the residue was purified by silica gel column chromatography (hexane:ethyl acetate = 5:1) to give the titled compound 90 mg (yield 52%) as an oil.
¹H-NMR δ(CDCl₃, 270MHz) 1.25 (3H, m), 1.55-2.15 (4H, m), 2.88-3.38 (3H, m), 3.66 (2H, m), 4.22 (2H, m), 4.91, 5.02 (total 1H, m), 7.13-7.32 (7H, m), 8.51 (1H, m), 8.63 (1H, m)

### (3) 5-Benzyl-6-(3-pyridyl)-2-azabicyclo[2. 2. 2]oct-5-ene

Three mL of aqueous 4N NaOH solution was added to a solution of 2-ethoxycarbonyl-5-benzyl-6-(3-pyridyl)-2-azabicyclo[2. 2. 2]oct-5-ene 90 mg (0.26 mmol) in 3 mL of methanol and the mixture was stirred in an autoclave at 150 °C for 6 hours. After methanol was evaporated in vacuo, the resultant aqueous layer was saturated with NaCl, extracted with chloroform. The extracts were dried over anhydrous Na₂SO₄, concentrated in vacuo and the residue was purified by silica gel column chromatography (chloroform : methanol : conc. aq. ammonia = 100 : 3 : 0.5) to give the titled compound 33 mg (yield 46%) as a pale yellow oil.
¹H-NMR δ(CDCl₃, 270MHz) 1.31 (1H, m), 1.65 (2H, m), 2.07 (1H, m), 2.50 (1H, m), 2.53 (1H, m), 2.94 (1H, d, J=8Hz), 3.62 (1H, d, J=5Hz), 3.67 (1H, d, J=5Hz), 3.81 (1H, t, J=3Hz), 7.15-7.31 (6H, m), 8.50 (1H, dd, J=2, 5Hz), 8.59 (1H, d, J=2Hz)

### Example 14

### 6-(2-Pyridyl)-2-azabicyclo[2. 2. 2]octane

### (1) 2-Ethoxycarbonyl-6-hydroxy-6-(2-pyridyl)-2-azabicyclo[2. 2. 2]octane

Under a nitrogen atmosphere, 1.3 mL (2 mmol) of a solution of 1.6 N n-butyl lithium in hexane was added dropwise to a solution of 2-bromopyridine 316 mg (2 mmol) in 6 mL of THF at -78 °C, and stirred for 15 min. To the mixture was added dropwise a solution of 2-ethoxycarbonyl-2-azabicyclo[2. 2. 2]octan-6-one 296 mg (1.5 mmol) in THF (3 mL) at the same temperature, and stirred for further 3 hours. After water was added and warmed to room temperature, the reaction mixture was extracted with ethyl acetate. The extracts were dried over anhydrous MgSO₄, concentrated in vacuo and the residue was purified by silica gel column chromatography (hexane:ethyl acetate = 3:1) to give the titled compound (the less polar isomer) 280 mg (yield 68%) as a brown oil.
¹H-NMR δ(CDCl₃, 270MHz) 0.76, 1.19 (total 3H, t, J=7Hz), 1.61-2.05 (4H, m), 2.19-2.52 (3H, m), 3.37, 3.50 (total 2H, m), 3.64 , 3.79 (total 1H, t, J=2.6Hz), 3.70, 4.00 (total 2H, m), 5.75 (1H, s), 7.15-7.24 (2H, m), 7.62-7.71 (1H, m), 8.52-8.55 (1H, m)

### (2) 2-Ethoxycarbonyl-6-chloro-6-(2-pyridyl)-2-azabicyclo[2. 2. 2]octane and 2-ethoxycarbonyl-6-(2-pyridyl)-2-azabicyclo[2. 2. 2]oct-5-ene

Thionyl chloride 5 mL was added to 2-ethoxylcarbonyl-6-hydroxy-6-(2-pyridyl)-2-azabicyclo[2. 2. 2]octane 220 mg (0.8 mmol) and the mixture was heated under reflux for 3.5 hours. After thionyl chloride was evaporated in vacuo and sat, aqueous sodium bicarbonate solution was added, the mixture was extracted with chloroform. The extracts were dried over anhydrous MgSO₄, concentrated in vacuo and the residue was purified by silica gel column chromatography (hexane:ethyl acetate = 5:1) to give 2-ethoxycarbonyl-6-chloro-6-(2-pyridyl)-2-azabicyclo[2. 2. 2]octane 120 mg (yield 53%) and 2-ethoxycarbonyl-6-(2-pyridyl)-2-azabicyclo[2. 2. 2]oct-5-ene 42 mg (yield 20%) respectively as oils.
¹H-NMR δ(CDCl₃, 270MHz)
Chloride; 1.00, 1.03 (total 3H, t, J=7Hz), 1.66 (1H, m), 1.82-2.05 (2H, m), 2.13-2.22 (2H, m), 2.63 (1H, m), 3.17-3.29 (2H, m), 3.69-3.91 (3H, m), 4.48 , 4.61 (total 1H, dd, J=2, 3.6Hz), 7.16 (1H, m), 7.69(1H, m), 7.75, 7.83 (total 1H, d, J=8Hz), 8.51 (1H, m)
Olefin; 1.23, 1.26 (total 3H, t, J=7Hz), 1.50-1.60 (2H, m), 1.74 (1H, m), 2.14 (1H, m), 2.97 (1H, br), 3.10 (1H, t, J=10Hz), 3.37 (1H, d, J=10Hz), 4.12 (2H, m), 5.60 (1H, m), 7.04-7.16 (2H, m), 7.49, 7.55 (total 1H, d, J=8Hz), 7.66 (1H, dt, J=2, 8Hz), 8.58 (1H, d, J=5Hz)

### (3) 2-Ethoxycarbonyl-6-(2-pyridyl)-2-azabicyclo[2. 2. 2]octane

A solution of 2-ethoxylcarbonyl-6-chloro-6-(2-pyridyl)-2-azabicyclo[2. 2. 2]octane 120 mg (0.42 mmol) in 10 mL of acetic acid was heated under reflux and 600 mg of zinc dust was added portionwise thereto. The mixture was stirred for further 10 min and cooled to room temperature, and acetic acid was evaporated in vacuo. Aqueous 1 N NaOH solution was added to the residue and the mixture was extracted with chloroform. The extracts were dried over anhydrous Na₂SO₄, concentrated in vacuo and the residue was purified by silica gel column chromatography (hexane:ethyl acetate = 1:1) to give the titled compound 50 mg (yield 46%) as a single isomer.
¹H-NMR δ(CDCl₃, 270MHz) 0.85, 1.88 (total 3H, t, J=7Hz), 1.72 (2H, m), 1.85-2.15 (5H, m), 3.25 (1H, m), 3.34 (1H, d, J=11Hz), 3.52, 3.60 (total 1H, d, J=11Hz), 3.78 ,4.02 (total 2H, q, J=7Hz), 4.09, 4.28 (total 1H, m), 7.10 (1H, m), 7.18, 7.28 (total 1H, d, J=8Hz), 7.60 (1H, t, J=8Hz), 8.52 (1H, m)

### (4) 6-(2-Pyridyl)-2-azabicyclo[2. 2. 2]octane

Under a nitrogen atmosphere, iodine 0.5 g (2 mmol) was added to hexamethyldisilane 1 mL (5 mmol) and the mixture was stirred at 100 °C for 15 min. After the mixture was allowed to cool to room temperature, a solution of 2-ethoxylcarbonyl-6-(2-pyridyl)-2-azabicyclo[2. 2. 2]octane 50 mg (0.19 mmol) in 1 mL of 1,2-dichloroethane was added and stirred at 55 °C for 3 hours. The reaction mixture was concentrated in vacuo and aq. 1 N NaOH solution was added to the residue. The mixture was extracted with chloroform, and the extracts were dried over anhydrous Na₂SO₄ and concentrated in vacuo to give the titled compound 32 mg (yield 90%) as a single isomer.
¹H-NMR δ(CDCl₃, 270MHz) 1.61-1.90 (4H, m), 2.02-2.15 (3H, m), 2.94 (1H, m), 3.00-3.16 (3H, m), 3.04 (1H, m), 7.13 (1H, dd, J=5, 7Hz), 7.17 (1H, d, J=7Hz), 7.61 (1H, t, J=7Hz), 8.56 (1H, d, J=5Hz)

### Example 15

### 6-(2-Pyridyl)-2-azabicyclo[2. 2. 2]oct-5-ene

Under a nitrogen atmosphere, iodine 0.5 g (2 mmol) was added to hexamethyldisilane 1 mL (5 mmol) and the mixture was stirred at 100 °C for 15 min. After the mixture was allowed to cool to room temperature, a solution of 2-ethoxylcarbonyl-6-(2-pyridyl)-2-azabicyclo[2. 2. 2]oct-5-ene 42 mg (0.16 mmol) obtained in Example 14(2) in 1 mL of 1,2-dichloroethane was added and stirred at 55 °C for 3 hours. The reaction mixture was concentrated in vacuo and aq. 1 N NaOH solution was added to the residue. The mixture was extracted with chloroform. The extracts were dried over anhydrous Na₂SO₄, concentrated in vacuo and the residue was purified by silica gel column chromatography (chloroform : methanol : conc. aq. ammonia = 100 : 2 : 0.2 → 100 : 10 : 0.2) to give the titled compound 5 mg (yield 17%) as a yellow oil.
¹H-NMR δ(CDCl₃, 270MHz) 1.38-1.53 (2H, m), 1.80 (1H, m), 2.22 (1H, m), 2.65 (1H, dt, J=3, 10.2Hz), 2.88 (1H, m), 3.13 (1H, d, J=10.2Hz), 3.61 (1H, br), 4.72 (1H,m), 7.02 (1H, dd, J=2, 7Hz), 7.14 (1H, dd, J=5, 7Hz), 7.50 (1H, d, J=7Hz), 7.65 (1H, t, J=7.6Hz), 8.54 (1H, d, J=5Hz)

### Example 16

### Syn-6-(5-pyrimidinyl)-2-azabicyclo[2. 2. 2]octane

### (1) 2-Ethoxycarbonyl-6-hydroxy-6-(5-pyrimidinyl)-2-azabicyclo[2. 2. 2]octane

Under a nitrogen atmosphere, 14 mL (23 mmol) of a solution of 1.6 N n-butyl lithium in hexane was added dropwise to a solution of 5-bromopyrimidine 3.58 g (22.5 mmol) in 100 mL of THF at -78 °C, and the mixture was stirred for 30 min. To the reaction mixture was added a solution of 2-ethoxycarbonyl-2-azabicyclo[2. 2. 2]octan-6-one 2.96 g (15 mmol) in 30 mL of THF dropwise at the same temperature, and the mixture was stirred for further 1 hour. After water was added and warmed to room temperature, the mixture was extracted with ethyl acetate. The extracts were washed with brine, dried over anhydrous MgSO₄, concentrated in vacuo and the residue was purified by silica gel column chromatography (chloroform:methanol = 30:1 → 20:1) to give the titled compound 1.99 g (yield 48%) as a brown oil.
¹H-NMR δ(CDCl₃, 270MHz) 0.84, 1.19 (total 3H, t, J=7Hz), 1.65-2.04 (4H, m), 2.17-2.39 (3H, m), 3.36 (2H, br), 3.78, 4.02 (total 2H, t, J=7Hz), 3.90, 4.04 (total 1H, br), 8.76 (2H, s), 9.01, 9.03 (total 1H, s)

### (2) Anti-2-ethoxycarbonyl-6-(5-pyrimidinyl)-2-azabicyclo[2. 2. 2]octane and syn-2-ethoxycarbonyl-6-(5-pyrimidinyl)-2-azabicyclo[2. 2. 2]octane

Ethyl chlorooxalate 0.91 mL (8.1 mmol) was added to a solution of 2-ethoxylcarbonyl-6-hydroxy-6-(5-pyrimidinyl)-2-azabicyclo[2. 2. 2]octane 1.50 g (5.4 mmol) and 4-dimethylaminopyridine 0.99 g (8.1 mmol) in 15 mL of acetonitrile and the mixture was stirred at room temperature for 1 hour. The reaction mixture was diluted with ethyl acetate, washed with water, sat, aqueous sodiumbicarbonate solution and then brine, dried over anhydrous Na₂SO₄ and concentrated in vacuo. The residue was dissolved in toluene 50 mL. Tributyltin hydride 2.3 mL (8.7 mmol) and 2,2'-azobisisobutyronitrile 100 mg was added there to and stirred at 100 °C for 1 hour. The solvent was evaporated in vacuo and the residue was purified by silica gel column chromatography (chloroform:acetenitrile = 7:1) to give *anti*-2-ethoxycarbonyl-6-(5-pyrimidinyl)-2-azabicyclo[2. 2. 2]octane 133 mg, *syn*-2-ethoxycarbonyl-6-(5-pyrimidinyl)-2-azabicyclo[2. 2. 2]octane 150 mg and a mixture of isomers 940 mg (yield in total 86%) respectively as a oil.
¹H-NMR δ(CDCl₃, 270MHz)
anti; 1.25-1.45 (3H, m), 1.55-2.00 (5H, m), 2.12, 2.28 (total 2H, m), 3.30-3.45 (1H, m), 3.49 (2H, br s), 4.01, 4.16 (total 1H, br s), 4.15-4.38 (2H, m), 8.69 (2H, d, J=5.0Hz), 9.11 (1H, d, J=3.3Hz)
syn; 0.90-1.02, 1.20-1.42 (total 3H, m), 1.52-2.35 (7H, m), 2.98-3.12 (1H, m), 3.40-3.65 (2H, m), 3.88-4.40 (3H, m), 8.58 (2H, d, J= 2.0Hz), 9.08 (1H, d, J=3.6Hz)

### (3) Syn-6-(5-pyrimidinyl)-2-azabicyclo[2. 2. 2]octane

*Syn*-2-ethoxycarbonyl-6-(5-pyrimidinyl)-2-azabicyclo[2. 2. 2]octane 310 mg (1.2 mmol) was subjected to removal of the ethoxycarbonyl group under the same condition as that in Example 14(4), and crude product so obtained was purified by silica gel column chromatography (chloroform:methanol:triethylamine = 100:10:1) to give the titled compound 129 mg (yield 59%) as a brown oil.
¹H-NMR δ(CDCl₃, 270MHz) 1.63-2.28 (7H, m), 2.80-3.25 (5H, m), 8.81 (2H, s), 9.08 (1H, s)

### Example 17

### Anti-6-(5-pyrimidinyl)-2-azabicyclol[2. 2. 2]octane

*Anti*-2-ethoxycarbonyl-6-(5-pyrimidinyl)-2-azabicyclo[2. 2. 2]octane 115 mg (0.44 mmol) obtained in Example 16(2) was subjected to removal of the ethoxycarbonyl group under the same condition as that in Example 14(4) and crude product so obtained was purified by silica gel column chromatography (chloroform:methanol:triethylamine = 100:10:1) to give the titled compound 37 mg (yield 44%) as a brown oil.
¹H-NMR δ(CDCl₃, 270MHz) 1.60-2.00 (5H, m), 2,10-2.30 (2H, m), 2.84 (1H, br s), 3.12 (2H, br s), 3.30-3.40 (1H, m), 8.68 (2H, s), 9.09 (1H, s)

### Example 18

### Syn-6-(3-methyl-5-isoxazolyl)-2-azabicyclo[2. 2. 2]octane

### (1) 2-Ethoxycarbonyl-6-hydroxy-6-methoxycarbonyl-2-azabicyclo[2. 2. 2]octane

To a mixture of water 20 mL and THF 20 mL were added 2-ethoxycarbonyl-2-azabicyclo[2. 2. 2]octan-6-one 3.0 g (15 mmol) and potassium cyanide 3.0g (45 mmol). An aqueous solution 10 mL of sodium metabisulfite 7.0 g (38 mmol) was added thereto with cooling in an ice bath. The reaction mixture was warmed to room temperature and stirred for further 1.5 hours. The organic layer was separated and the aqueous layer was extracted with ethyl acetate. The organic layers were combined, washed with brine; dried over anhydrous MgSO₄ and concentrated in vacuo. The residue was dissolved in methanol 20 mL, and was added dropwise to sat. hydrogen chloride solution in ether (80 mL) and methanol (80 mL) at -78 °C. The mixture was warmed to 0 °C and left at the same temperature for 15 hours. The reaction mixture was concentrated and water 10 mL was added thereto. The mixture was stirred at room temperature for 1 hour, extracted with ether. The extracts were washed with brine, dried over anhydrous MgSO₄, concentrated in vacuo and the residue was purified by silica gel column chromatography (hexane:ethyl acetate = 1:1) to give the titled compound 3.7 g (yield 96%) as a pale yellow oil.
¹H-NMR δ(CDCl₃, 270MHz) 1.15-1.32 (3H, m), 1.50-1.95 (4H, m), 2.20-2.57 (3H, m), 3.15-3.50 (2H, m), 3.70-3.82 (3H, m), 3.85-4.22 (3H, m)

### (2) 2-Ethoxycarbonyl-6-hydroxy-6-(3-methyl-5-isoxazolyl)-2-azabicyclo[2. 2. 2]octane

Under a nitrogen atmosphere, to a solution of acetoxime 2.65 g (36.3 mmol) in 30 mL of THF was added dropwise 57 mL (90 mmol) of a solution of 1.6 N n-butyl lithium in hexane with cooling in an ice bath, and stirred at the same temperature for 1 hour. The mixture was cooled to -78 °C and a solution of 2-ethoxycarbonyl-6-hydroxy-6-methoxycarbony-2-azabicyclo[2. 2. 2]octane 3.7 g (14 mmol) in THF (20 mL) was added thereto and stirred for further 20 min. After warmed to room temperature, to the mixture was added water 10 mL and conc. sulfuric acid 5 ml, and the mixture was heated under reflux for 2 hours. After allowed to cool to room temperature, the organic layer was separated and the aqueous layer was extracted with ethyl acetate. The organic layers were combined, washed with brine, dried over anhydrous MgSO₄, concentrated in vacuo and the residue was purified by silica gel column chromatography (hexane:ethyl acetate = 1:1) to give the titled compound 1.53 g (yield 38%) as white solid.
¹H-NMR δ(CDCl₃, 270MHz) 1.05-1.35 (3H, m), 1.50-2.45 (10 H, m), 3.20-3.65 (2H, m), 3.88-4.32 (3H, m), 5.98, 6.14 (total 1H, s)

### (3) 2-Ethoxycarbonyl-6-chloro-6-(3-methyl-5-isoxazolyl)-2-azabicyclo[2. 2. 2]octane

Five mL of thionyl chloride was added dropwise to a solution (10 mL) of 2-ethoxylcarbonyl-6-hydroxy-6-(3-methyl-5-isoxazolyl)-2-azabicyclo[2. 2. 2]octane 1.08 g (3.86 mmol) in 10 mL of methylene chloride with cooling in an ice bath. The mixture was warmed to room temperature and stirred for 4 hours. The reaction mixture was concentrated under reduced pressure. Toluene was added to the residue, and evaporeted again under reduced pressure to give the titled compound 1.08 g (yield 94%) as a brown oil.
¹H-NMR δ(CDCl₃, 270MHz) 1.15-1.35 (3H, m), 1.55-2.90 (7H, m), 2.27 (3H, s), 3.20-3.35 (2H, m), 3.92-4.25 (2H, m), 4.50-4.58 (1H, m), 6.11, 6.30 (total 1H, s)

### (4) Anti-2-ethoxycarbonyl-6-(3-methyl-5-isoxazolyl)-2-azabicyclo[2. 2. 2]octane and syn-2-ethoxycarbonyl-6-(3-methyl-5-isoxazolyl)-2-azabicyclo[2. 2. 2]octane

10 % Palladium/carbon 310 mg was added to a solution of 2-ethoxycarbonyl-6-chloro-6-(3-methyl-5-isoxazolyl)-2-azabicyclo[2. 2. 2]octane 880 mg (2.95 mmol) in 10 mL of ethyl acetate and the mixture was stirred under hydrogen atmosphere for 1 hour. After the catalyst was filtered off through Celite® bed, the filtrate was concentrated in vacuo, and the residue was purified by silica gel column chromatography (hexane:ethyl acetate = 3:1 →. 2:1) to give *anti*-2-ethoxycarbonyl-6-(3-methyl-5-isoxazolyl)-2-azabicyclo[2. 2. 2]octane (less polar isomer) 178 mg, *syn*-2-ethoxycarbonyl-6-(3-methyl-5-isoxazolyl)-2-azabicyclo[2. 2. 2]octane (more polar isomer) 236 mg and mixture of isomers 209 mg (yield in total 71%) respectively as a oil.
¹H-NMR δ(CDCl₃, 270MHz)
anti isomer; 1.28 (3H, t, J=6.9Hz), 1.55-2.20 (7H, m), 2.28, 2.29 (total 3H, s), 3.33-3.50 (1H, m), 3.43 ( 2H, br s), 4.12-4.37 (3H, m), 5.91, 5.93 (total 1H, s)
syn isomer; 1. 10, 1.24 (total 3H, t, J=6.9Hz), 1.60-2.20 (7H, m), 2.24 (3H, s), 3.12-3.23 (1H, m), 3.30-3.50 (2H, m), 3.90-4.37 (3H, m), 5.85, 6.00 (total 1H, s)

### (5) Syn-6-(3-methyl-5-isoxazolyl)-2-azabicyclo[2. 2. 2]octane

*Syn*-2-ethoxycarbonyl-6-(3-methyl-5-isoxazolyl)-2-azabicyclo[2. 2. 2]octane 190 mg (0.72 mmol) was subjected to removal of the ethoxycarbonyl group under the same condition as that in Example 14(4), and crude product so obtained was purified by silica gel column chromatography (chloroform:methanol:triethylamine = 100:10:1) to give the titled compound 140 mg (yield quantitative) as a brown oil.
¹H-NMR δ(CDCl₃, 270MHz) 1.60-2.20 (7H, m), 2.29 (3H, s), 3.01 (3H, br s), 3.14 (1H, dd, J=6.3, 11Hz), 5.96 (1H, s)

### Example 19

### Anti-6-(3-methyl-5-isoxazolyl)-2-azabicyclo[2. 2. 2]octane

*Anti*-2-ethoxycarbonyl-6-(3-methyl-5-isoxazolyl)-2-azabicyclo[2. 2. 2]octane 175 mg (0.66 mmol) obtained in Example 18(4) was subjected to removal of the ethoxycarbonyl group under the same condition as that in Example 14(4), and crude product so obtained was purified by silica gel column chromatography (chloroform:methanol:triethylamine = 100:10:1) to give the titled compound 89 mg (yield 70%) as a brown oil.
¹H-NMR δ(CDCl₃, 270Mhz) 1.60-1.95 (6H, m), 2.14-2.30 (1H, m), 2.28 (3H, s), 3.09 (2H, br s), 3.42 (1H, br t, J=7Hz), 5.89 (1H, s)

### Example 20

### 6-(3-Methyl-5-isoxazolyl)-2-azabicyclo[2. 2. 2]oct-5-ene

### (1) 2-Ethoxycarbonyl-6-(3-methyl-5-isoxazolyl)-2-azabicyclo[2. 2. 2]oct-5-ene

Two mL of DBU was added to 2-ethoxycarbonyl-6-chloro-6-(3-methyl-5-isoxazolyl)-2-azabicyclo[2. 2. 2]octane 200 mg (0.67 mmol) obtained in Example 18(3) . The mixture was stirred at 130 °C for 6 hours and allowed to cool to room temperature. Water was added thereto, and the mixture was extracted with ether. The extracts were washed with 1 N hydrochloric acid and brine, dried over anhydrous MgSO₄, concentrated in vacuo, and the residue was purified by silica gel column chromatography (hexane:ethyl acetate = 2:1) to give the titled compound 125 mg (yield 71%) as a yellow oil.
¹H-NMR δ(CDCl₃, 270MHz) 1.20-1.32 (3H, m), 1.42-1.80 (3H, m), 2.02-2.20 (1H, m), 2.24, 2.31 (total 3H, s), 2.90-3.17 (2H, m), 3.36 (1H, dd, J=2.0, 10Hz), 4.05-4.20 (2H, m), 5.04, 5.11 (total 1H, br s), 6.08, 6.19 (total 1H, s), 6.95 (1H, dd, J=1.6, 6.6Hz)

### (2) 6-(3-Methyl-5-isoxazolyl)-2-azabicyclo[2. 2. 2]oct-5-ene

2-Ethoxycarbonyl-6-(3-methyl-5-isoxazolyl)-2-azabicyclo[2. 2. 2]oct-5-ene 120 mg (0.46 mmol) was subjected to removal of the ethoxycarbonyl group under the same condition as that in Example 14(4), and crude product so obtained was purified by silica gel column chromatography (chloroform:methanol:triethylamine = 100:10:1) to give the titled compound 75 mg (yield 86%) as a brown oil.
¹H-NMR δ(CDCl₃, 270MHz) 1.30-1.52 (2H, m), 1.69-1.80 (1H, m), 2.00-2.15 (2H, m), 2.29 (2H, s), 2.56 (1H, dt, J=10, 3.0Hz), 2.75-2.83 (1H, m), 3.04 (1H, dd, J=1.7, 10Hz), 3.88-3.97 (1H, m), 6.04 (1H, s), 6.95 (1H, d, J=6.9Hz)

### Example 21

### Syn-2-methyl-6-(3-methyl-5-isoxazolyl)-2-azabicyclo[2. 2. 2.]octane

*Syn*-6-(3-methyl-5-isoxazolyl)-2-azabicyclo[2. 2. 2]octane 75 mg (0.39 mmol) obtained in Example 18 was dissolved in a mixture of 37 % formal in 1 mL and formic acid 1 mL. The mixture was heated under reflux for 1 hour, concentrated in vacuo and aq. 1 N NaOH solution was added thereto. The mixture was extracted with ether, and the extracts were dried over anhydrous Na₂SO₄, concentrated in vacuo, and the residue was purified by silica gel column chromatography (chloroform:methanol:triethylamine = 300:10:1) to give the titled compound 62 mg (yield 89%) as a colorless oil.
¹H-NMR δ(CDCl₃, 270MHz) 1.45-1.83 (5H, m), 2.00-2.17 (2H, m), 2.25-2.35 (1H, m), 2.26 (3H, s). 2.28 (3H, s), 2.77 (1H, br s), 2.99 (1H, br t, J=10Hz), 3.07 (1H, td, J=2, 10Hz), 5.95 (1H, s)

### Example 22

### 6-(3-Methyl-5-isoxazolyl)-6-(2-propyn-1-oxy)-2-azabicyclo[2. 2. 2]octane

### (1) 2-Ethoxycarbonyl-6-(3-methyl-5-isoxazolyl)-6-(2-propyn-1-oxy)-2-azabicyclo[2. 2. 2]octane

Under a nitrogen atmosphere, to a suspension of 60 % NaH 16 mg (0.39 mmol) in 2 mL of THF was added a solution of 2-ethoxycarbonyl-6-hydroxy-6-(3-methyl-5-isoxazolyl)-2-azabicyclo[2. 2. 2]octane 100 mg (0.36 mmol) obtained in Example 18(2) in 2 mL of THF with cooling in an ice bath, and stirred for 1 hour. Propargyl bromide 42 µL (0.59 mmol) was added, and stirred at the same temperature for 40 min, at room temperature for 5 hours and at 50 °C for further 1 hour. After allowed to cool to room temperature, the reaction mixture was diluted with ethyl acetate, washed with water and brine, dried over anhydrous Na₂SO₄, concentrated in vacuo, and the residue was purified by silica gel column chromatography (hexane:ethyl acetate = 2:1) to give the titled compound 46 mg (yield 41%: mixture of isomers ca. 1:1) as a brown oil.
¹H-NMR δ(CDCl₃, 270MHz) 1.14-1.33 (3H, m), 1.34-2.42 (11H, m), 3.13-3.55 (2H, m), 3.80-4.61 (5H, m), 6.13, 6.16, 6.19, 6.36 (total 1H, s)

### (2) 6-(3-Methyl-5-isoxazolyl)-6-(2-propyn-1-oxy)-2-azabicyclo[2. 2. 2]octane

2-Ethoxycarbonyl-6-(3-methyl-5-isoxazolyl)-6-(2-propyn-1-oxy)-2-azabicyclo[2. 2. 2]octane 45 mg (0.14 mmol) was subjected to removal of the ethoxycarbonyl group under the same condition as that in Example 14(4), and crude product so obtained was purified by silica gel column chromatography (chloroform:methanol:triethylamine = 100:10:1) to give the titled compound 26 mg (yield 75%: mixture of isomers ca. 1:1) as an oil.
¹H-NMR δ(CDCl₃, 270MHz) 1.23-2.43 (8H, m), 2.32, 2.33 (total 3H, s), 2.80-3.32 (3H, m), 3.82-4.10 (2H, m), 6.17, 6.21 (total 1H, s)

### Example 23

### 6-(2-Pyrazinyl)-2-azabicyclo[2. 2. 2]oct-5-ene

### (1) 2-Ethoxycarbonyl-6-hydorxy-6-(2-pyrazinyl)-2-azabicyclo[2. 2. 2]octane

1.9 Milliliter(3 mmol) of a solution of 1.6 N n-butyl lithium in hexane was added dropwise, under a nitrogen atmosphere, to a solution of 2-iodopyrazine 0.62 g (3 mmol: JP 64-26567-A) in 7 mL of THF at -100 °C, and stirred for 10 min. To the mixture was added a solution of 2-ethoxycarbonyl-2-azabicyclo[2. 2. 2]octan-6-one 0.30 g (1.5 mmol) in THF (3 mL) at the same temperature, and the mixture was warmed to -78 °C and stirred for further 2 hours. After water was added and warmed to room temperature, the mixture was extracted with ethyl acetate. The extracts were washed with brine, dried over anhydrous MgSO₄, concentrated in vacuo and the residue was purified by silica gel column chromatography (hexane:ethyl acetate = 1:1) to give the titled compound 308 mg (yield 74%) as a yellow oil.
¹H-NMR δ(CDCl₃, 270MHz) 0.78, 1.19 (total 3H, 1H, J=7Hz), 1.67-1.84 (3H, m), 2.05 (1H, m), 2.25 (1H, m), 2.36-2.50 (2H, m), 3.38 (1H, m), 3.53 (1H, m), 3.70-3.83 (2H, m), 4.00 (1H, m), 4.75, 4.92 (total 1H, s), 8.50-8.61 (3H, m)

### (2) 2-Ethoxycarbonyl-6-(2-pyrazinyl)-2-azabicyclo[2. 2. 2]oct-5-ene

2-Ethoxycarbonyl-6-hydroxy-6-(2-pyrazinyl)-2-azabicyclo[2. 2. 2]octane 100 mg (0.36 mmol) was subjected to the same reaction as that in Example 18(3) to give the corresponding chloride, followed by the same reaction as that in Example 20(1) to give the titled compound 59 mg (yield 63%) as an oil.
¹H-NMR δ(CDCl₃, 270MHz) 1.20-1.32 (3H, m), 1.45-1.64 (2H, m), 1.70-1.85 (1H, m), 2.10-2.25 (1H, m), 3.01 (1H, br s), 3.08-3.20 (1H, m), 3.40 (1H, dd, J=10, 2.0Hz), 4.05-4.20 (1H, m), 5.61, 5.72 (total 1H, br s), 7.12-7.22 (1H, m), 8.41 (1H, br s), 8.53 (1H, d, J=2.3Hz), 8.82 (1H, br d, J=4.3Hz)

### (3) 6-(2-Pyrazinyl)-2-azabicyclo[2. 2. 2]oct-5-ene

2-Ethoxycarbonyl-6-(2-pyrazinyl)-2-azabicyclo[2. 2. 2]oct-5-ene 30 mg (0.12 mmol) was subjected to the same react inn as that in Example 7(3) to give the titled compound 9 mg (yield 40%) as a brown oil.
¹H-NMR δ(CDCl₃, 270MHz) 1.33-1.55 (2H, m), 1.73-1.85 (1H, m), 2.05-2.20 (1H, m), 2.61 (1H, dt, J=10, 2.0Hz), 2.82-2.90 (1H, m), 3.08 (1H, dd, J=10, 1.7Hz), 4.52 (1H, t, J=2.3Hz), 7.13 (1h, dd, J=6.9, 2.0Hz), 8.39 (1H, d, J=2.6Hz), 8.49 (1H, t, J=1.7Hz), 8.80 (1H, d, J=1.0Hz)

### Example 24

### 5-(5-Bromo-3-pyridyl)-2-azabicyclo[2. 2. 2]oct-5-ene

### (1) 2-Ethoxycarbonyl-6-hydorxy-6-(5-bromo-3-pyridyl)-2-azabicyclo[2. 2. 2]octane

0.48 Milliliter(0.76 mmol) of a solution of 1.6 N n-butyl lithium in hexane was added dropwise at -100 °C, under a nitrogen atmosphere, to a solution of 3,5-dibromopyridine 240 mg (1 mmol) in 10 mL of THF, and stirred for 5 min. To the mixture was added a solution of 2-ethoxycarbonyl-2-azabicyclo[2. 2. 2]octan-6-one 100 mg (0.51 mmol) in 3 mL of THF at the same temperature, and the mixture was warmed to -78 °C over 1 hour. After sat. aq. NH₄Cl solution was added and warmed to room temperature, the mixture was extracted with ethyl acetate. The extracts were washed with brine, dried over anhydrous MgSO₄, concentrated in vacuo and the residue was purified by silica gel column chromatography (chloroform:methanol = 30:1) to give the titled compound 129 mg (yield 71%) as a pale yellow oil.
¹H-NMR δ(CDCl₃, 270MHz) 0.90, 1.20 (total 3H, t, J=7.0 Hz), 1.60-2.05 (4H, m), 2.15-2.45 (3H, m), 2.96, 3.16 (total 1H, br s), 3.28-3.45 (2H, m), 3.72-4.10 (3H, m), 7.94 (1H, dt, J= 9.9 Hz), 8.45-8.60 (2H, m)

### (2) 2-Ethoxycarbonyl-6-(5-bromo-3-pyridyl)-2-azabicyclo[2. 2. 2]oct-5-ene

One mL of thionyl chloride was added to a solution of 2-ethoxycarbonyl-6-hydroxy-6-(5-bromo-3-pyridyl)-2-azabicyclo[2. 2. 2]octane 55 mg (0.51 mmol) in 1 mL of methylene chloride, and the mixture was stirred at room temperature for 1.5 hours. The reaction mixture was concentrated. After pyridine 1 mL was added to the residue, the mixture was stirred at 100 °C for 2 hours and under reflux for further 7 hours. After allowed to cool to room temperature and pyridine was distilled off, the residue was purified by silica gel chromatography (hexane:ethyl acetate = 2:1) to give the titled compound 51 mg (yield 98%) as a colorless oil.
¹H-NMR δ(CDCl₃, 270MHz) 1.22-1.40 (3H, m), 1.45-1.60 (2H, m), 1.70-1.80 (1H, m), 2.05-2.20 (1H, m), 2.90-3.03 (1H, m), 3.04-3.20 (1H, m), 3.37 (1H, br d, J= 9.9Hz), 4.10-4.25 (2H, m), 5.12, 5.24 (total 1H, br s), 6.72 (1H, br d, J= 6.6Hz), 7.91 (1H, br d, J=10Hz), 8.54-8.70 (2H, m)

### (3) 6-(5-Bromo-3-pyridyl)-2-azabicyclo[2. 2. 2]oct-5-ene

The titled compound 32mg (yield 82%) was obtained as an oil by removal of the ethoxycarhonyl group from 2-ethoxycarbonyl-2-azabicyclo[2. 2. 2]octan-6-one 50 mg (0.15 mmol) under the same condition as that in Example 14(4).
¹H-NMR δ(CDCl₃, 270Mhz) 1.24-1.60 (2H, m), 1.82 (1H, br t, J=10Hz), 2, 27 (1H, br t, J=9.6Hz), 2.70 (1H, br d, J=10Hz), 2.92 (1H, br d, J=7.3 Hz), 3.16 (1H, br d, J=10Hz), 4.32 (1H, br s), 6.81 (1H, dd, J= 6.9, 1.7Hz), 7.91 (1H, t, J=2.0Hz), 8.55 (1H, d, J=1.3Hz), 8.61 (1H, d, J=2.0Hz)

### Example 25

### Anti-5-(2-pyrazinyl)-2-azabicyclo[2. 2. 2]octane

### (1) 2-Ethoxycarhonyl-5-hydroxy-5-(2-pyrazinyl)-2-azabicyclo[2. 2. 2]octane

3.1 Milliliter(5 mmol) of a solution of 1.6 N n-hutyl lithium in hexane was added dropwise at -100 °C, under a nitrogen atmosphere, to a solution of 2-iodopyrazine 1.03 g (5 mmol) in 10 mL of THF, and stirred for 10 min. To the mixture was added a solution of 2-ethoxycarbonyl-2-azabicyclo[2. 2. 2]octan-5-one 0.49 g (2.5 mmol) in 3 mL of THF at the same temperature, and the mixture was warmed to -78 °C and stirred for further 2 hours. After water was added and warmed to room temperature, the mixture was extracted with ethyl acetate. The extracts were washed with brine, dried over anhydrous MgSO₄, concentrated in vacuo and residue was purified by silica gel column chromatography (hexane:ethyl acetate = 1:2) to give the titled compound 405 mg (yield 58%) as a yellow oil.
¹H-NMR δ(CDCl₃, 270MHz) 1.26 (3H, m), 1.55-2.10, 2.50(total 6H, m), 2.80 (1H, m), 3.12, 3.26, 4.13 (total 4H, m), 4.30, 4.41 (total 1H, m), 8.54 (2H, m), 8.75, 8.88 (total 1H, br)

### (2) Anti-2-ethoxycarbonyl-5-(2-pyrazinyl)-2-azabicyclo[2. 2. 2]octane and syn-2-ethoxycarbonyl-6-hydroxy-5-(2-pyrazinyl)-2-azabicyclo[2. 2. 2]octane

2-Ethoxycarbonyl-5-hydroxy-5-(2-pyrazinyl)-2-azabicyclo[2. 2. 2]octane 265 mg (0.96 mmol) was subjected to the sane reaction as that in Example 16(2) to give the titled *anti*-isomer 72 mg, *syn*-isomer 58 mg and mixture of the isomers 58 mg (yield in total 75%) respectively as an oil.
¹H-NMR δ(CDCl₃, 270MHz)
anti isomer: 0.87-2.00 (7H, m), 2.10-2.42 (3H, m), 3.27- 3.38 (1H, m), 3.49 (1H, dd, J= 11, 2.6 Hz), 3.60-3.70 (1H, m), 4.10-4.35 (3H, m), 8.42 (1H, d, J= 2.6 Hz), 8.50-8.58 (2H, m)
syn isomer: 1.20-2.60 (10H, m), 3.20-3.52 (3H, m), 4.08-4.35 (3H, m), 8.38-8.42 (1H, m), 8.50-8.57 (2H, m)

### (3) Anti-5-(2-pyrazinyl)-2-azabicyclo[2. 2. 2]octane

The titled compound 27 mg (yield 53%) was obtained as white solid from *anti*-2-ethoxycarbonyl-5-(2-pyrazinyl)-2-azabicyclo[2. 2. 2]octane 70 mg (0.27 mmol) by carrying out the same reaction as that in Example 14(4).
¹H-NMR δ(CDCl₃, 270MHz) 1.20-2.00 (6H, m), 1.94 (1H, br s), 2.18-2.42 (2H, m), 3.11 (1H, br s), 3.20 (1H, dd, J= 11, 2.3Hz), 3.28 (1H, dt, J= 11, 2.7 Hz), 3.35-3.45 (1H, m), 8.41 (1H, d, J=1.7Hz), 8.54 (2H, d, J= 1.7Hz)

### Example 26

### Syn-5-(2-pyrazinyl)-2-azabicyclo[2. 2. 2]octane

The titled compound 14 mg (yield 33%) was obtained from *syn*-2-ethoxycarbonyl-5-(2-pyrazinyl)-2-azabicyclo[2. 2. 2]octane 58 mg (0.22 mmol) by carrying out the same reaction as that in Example 14(4).
¹H-NMR δ(CDCl₃, 270MHz) 1.70-2.08 (6H, m), 2.42-2.52 (1H, m), 2.80 (1H, dt, J= 11.5, 1.9Hz), 2.98 (1H, dt, J= 11.5, 2.3Hz), 3.01-3.07 (1H, m), 3.24-3.32 (1H, m), 8.41 (1H, d, J= 2.6Hz), 8.50-8.60 (2H, m)

### Example 27

### 5-(2-Pyrazinyl)-2-azabicyclo[2. 2. 2]oct-5-ene

### (1) 2-Ethoxycarbonyl-5-(2-pyrazinyl)-2-azabicyclo[2. 2. 2]oct-5-ene

Thionyl chloride 1 mL was added to a solution of 2-ethoxycarbonyl-5-hydroxy-5-(2-pyrazinyl)-2-azabicyclo[2. 2. 2]octane 100 mg (0.36 mmol) obtained in Example 25(1) in 1 mL of methylene chloride, and the mixture was stirred at room temperature for 4 hours. After concentrated under reduced pressure, DBU 0.5 mL was added and the mixture was stirred at 130 °C for 2. hours. After allowed to cool to room temperature, the mixture was diluted with water and extracted with ether. The extracts were washed with brine, dried over anhydrous Na₂SO₄ and concentrated under reduced pressure, and the residue was purified by silica gel column chromatography (hexane:ethyl acetate = 1:1) to give the titled compound 54 mg (yield 58%) as a yellow oil.
¹H-NMR δ(CDCl₃, 270MHz) 1.70-2.07 (6H, m), 2.41-2.52 (1H, m), 2.80 (1H, dt, J= 11.5, 2.0Hz), 2.98 (1H, dt, J= 11.5, 2.3Hz), 3.01-3.06 (1H, m). 3.24-3.32 (1H, m), 8.41 (1H, d, J= 2.6Hz), 8.52-8.59 (2H, m)

### (2) 5-(2-Pyrazinyl)-2-azabicyclo[2. 2. 2]oct-5-ene

The titled compound 21 mg (yield 65%) was obtained as a yellow oil by removal of the ethoxycarbonyl group from 2-ethoxycarbonyl-5-(2-pyrazinyl)-2-azabicyclo[2. 2. 2]oct-5-ene 45 mg (0.17 mmol) by the same method as that in Example 7(3), followed by purification of so obtained crude product by silica gel column chromatography (chloroform: methanol : conc. aq. ammonia solution = 100 : 10 : 3).
¹H-NMR δ(CDCl₃, 270MHz) 1.33-1.53 (2H, m), 1.76-1.87 (1H, m), 2.02-2.12 (1H, m), 2.60 (1H, dt, J=10, 2.8Hz), 3.10 (1H, dd, J=10, 2.0 Hz), 3.55-3.62 (1H, m), 3.72-3.78 (1H, m), 7.25 (1H, dd, J=5.9, 1.6Hz), 8.39 (1H, d, J=2.6Hz), 8.51 (1H, dd, J=2.6, 1.6Hz), 8.82 (1H, d, J=1.7Hz)

### Example 28

### Syn-6-(5-ethynyl-3-pyridyl)-2-azabicyclo[2. 2. 2]octane

### (1) Syn-2-ethoxycarbonyl-6-(5-bromo-3-pyridyl)-2-azabicyclo[2. 2. 2]octane

2-Ethoxycarbonyl-6-(5-bromo-3-pyridyl)-2-azabicyclo[2. 2. 2]oct-5-ene 880 mg (2.6 mmol) obtained in Example 24(2) and benzene sulfonyl hydrazine 1.34 g (7.8 mmol) in toluene 10 mL were stirred under reflux for 8 hours. After the mixture was allowed to cool and concentrated, the residue was purified by silica gel chromatography (hexane:ethyl acetate = 2:1) to give the titled compound(less polar isomer) 290 mg (yield 33%) as an oil.
¹H-NMR δ(CDCl₃, 270MHz) 0.99 and 1.23 (total 3H, t, J= 6.9 Hz), 1.58-2.30 (7H, m), 2.98-3.10 (1H, m), 2.35-2.60 (2H, m), 3.80-4.20 (3H, m), 7.64-7.68 (1H, m), 8.38 (1H, d, J= 2 Hz), 8.51 and 8.53 (total 1H, d, J= 2 Hz).

### (2) Syn-2-( tert-butoxycarbonyl )-6-(5-bromo-3-pyridyl)-2-azabicyclo[2. 2. 2]octane

Iodine 1.00 g (3.94 mmol) and hexamethyldisilane 1.0 mL (4.9 mmol) were mixed and stirred at 100 °C for 10 min. After allowed to cool, a solution of *syn*-2-ethoxycarbonyl-6-(5-bromo-3-pyridyl)-2-azabicyclo[2. 2. 2]octane 290 mg (0.86 mmol) in 5 mL of 1,2-dichloroethane was added and stirred at 50 °C for 2 hours. Methanol 1 mL was added and the reaction mixture was concentrated in vacua. Sat. aq. NaHCO₃ solution and aq. Na₂S₂O₃ solution were added thereto and the mixture was extracted with chloroform, dried over anhydrous Na₂SO₄ and concentrated in vacuo. The residue was dissolved in methylene chloride 3 mL, and triethylamine 0.18 mL (1.3 mmol) and di-*tert*-butyl dicarbonate 293 mg (1.3 mmol) was added thereto, and stirred at room temperature for 2 hours. The reaction mixture was concentrated in vacua and the residue was purified by silica gel chromatography (hexane:ethyl. acetate = 3:1) to give the titled compound 279 mg (yield 89%) as a oil.
¹H-NMR δ(CDCl₃, 270MHz) 1.65-2.40 (7H, m), 3.09 (1H, dd, J=11, 7.5 Hz), 3.21 (1H, d, J= 11 Hz), 3.24-3.35 (2H, m), 7.94 (1H, dd, J=2.3, 2.0 Hz), 8.51 (1H, d, J= 2.3 Hz), 8.54 (1H, d, J= 2.0 Hz). SSG-659; 1.24 and 1.44 (total 9H, s), 1.50-2.25 (7H, m), 2.99-3.08 (1H, m), 3.32-3.51 (2H, m), 3.91 and 4.08 (total 1H, br s), 7.68 (1H, br s), 8.38 (1H, dd, J= 7.3, 2.0 Hz), 8.52 (1H, dd, J= 9.6, 2.3 Hz).

### (3) Syn-2-( tert-butoxycarbonyl)-6-(5-(3-hydroxy-3-methyl-1-butynyl)-3-pyridyl)-2-azabicyclo[2. 2. 2]octane

To a mixture of 1,2-dimethoxyethane 4 mL and water 4 mL were added *syn*-2-( *tert*-butoxycarbanoyl )-6-(5-bromo-3-pyridyl )-2-azabicyclo[2. 2. 2]octane 220 mg (0.55 mmol), 10 % palladium/carbon 23 mg, triphenylphosphine 23 mg, copper(I) iodide 8 mg and potassium carbonate 188 mg (1.4 mmol), and the mixture was stirred for 45 min. 2-Methyl-3-butyn-2-ol 115 mg (1.37 mmol) was added thereto and stirred at 80 °C for 11 hours. After allowed to cool, insoluble substances were filtered off and the filtrate was extracted with ethyl acetate. The extracts were washed with brine, dried over anhydrous Na₂SO₄, concentrated in vacuo and the residue was purified by silica gel chromatography (hexane:ethyl acetate = 1:1) to give the titled compound 185 mg (yield 92%) as an oil.
¹H-NMR δ(CDCl₃, 270MHz) 1.24 and 1.44 (total 9H, s), 1.60 (6H, s), 1.50-2.23 (7H, m), 2.89 (1H, br s, -OH), 2.93-3.10 (1H, m), 3.30-3.53 (2H, m), 3.90 and 4.04 (total 1H, br s), 7.55 (1H, br s), 8.35 (1H, s), 8.55 (1H, s).

### (4) Syn-2-(tert-butoxycarbonyl)-6-(5-ethynyl-3-pyridyl)-2-azabicyclo[2. 2. 2]octane

To a suspension of 60 % NaH 22 mg (0.55 mmol) in 1 mL of toluene was added a solution of *syn*-2-(*tert*-butoxycarbonyl)-6-(5-(3-hydroxy-3-methyl-1-butynyl)-3-pyridyl)-2-azabicyclo[2. 2. 2]octane 180 mg (0.49 mmol) in 2 mL of toluene, and the mixture was stirred at room temperature for 30 min and at 80 °C to refluxing temperature for 5.5 hours. After allowed to cool, the mixture was diluted with ethyl acetate, washed with water and brine, dried over anhydrous Na₂SO₄, concentrated in vacuo, and the residue was purified by silica gel chromatography (hexane:ethyl acetate = 1:1) to give the titled compound 89 mg (yield 59%) as an oil.
¹H-NMR δ(CDCl₃, 270MHz) 1.23 and 1.43 (total 9H, s), 1.60-2.23 (7H, m), 3.02 (1H, dd, J= 10, 6.9 Hz), 3.15 and 3.18 (total 1H, s), 3.32-3.42 (1h, m), 3.45-3.53 (1H, m), 3.90 and 4.05 (total 1H, br s), 7.58-7.64 (1H, m), 8.41 (1H, dd, J= 5.6, 2.3 Hz), 8.56 (1h, dd, J= 8.3, 2.0 Hz).

### (5) Syn-6-(5-ethynyl-3-pyridyl)-2-azabicyclo[2. 2. 2]octane

One mL trifluoroacetic acid was added to a solution of *syn*-2-(*tert*-butoxycarbonyl)-6-(5-ethynyl-3-pyridyl)-2-azabicyclo[2. 2. 2]octane 75mg (0.24 mmol) in 2 mL of methylene chloride, and stirred at room temperature for 40 min. After concentration in vacuo, aq. 10% K₂CO₃ solution was added thereto. The mixture was extracted with chloroform, and the extracts were dried over anhydrous Na₂SO₄ and concentrated in vacuo. The residue was purified by silica gel chromatography (chloroform : methanol : conc. aq. ammonia solution = 100 : 10 : 1) to give the titled compound 59 mg (yield quantitative) as an oil.
¹H-NMR δ(CDCl₃, 270MHz) 1.60-2.17 (7H, m), 2.71 (1H. td. J=3.0, 1.8Hz), 3.02 (1H, dd, J= 11, 7.3 Hz), 3.08 (2H, s). 3.20 (1H, s), 7.86 (1H, t like, J= 2 Hz), 8.52 (1H, d, J= 2.2 Hz), 8.57 (1H, d, J= 2.0 Hz).

### Example 29

### Syn-6-(5-phenyl-3-pyridyl)-2-azabicyclo[2. 2. 2]octane

### (1) Syn-2-ethoxycarbonyl-6-(5-phenyl-3-pyridyl)-2-azabicyclo[2. 2. 2]octane

To a mixture of 1,2-dimethoxyethane 2 mL and water 2 mL were added *syn*-2-ethoxycarbonyl-6-(5-bromo-3-pyridyl)-2-azabicyclo[2. 2. 2]octane 100 mg (0.3 mmol), phenylboronic acid 936 mg (0.3 mmol), tetrakistriphenylphosphine palladium 17 mg and sodium carbonate 103 mg (0.97 mmol), and stirred under reflux for 2.5 hours. After allowed to cool, the mixture was diluted with ethyl acetate, washed with water and brine, dried over anhydrous Na₂SO₄, concentrated in vacuo and the residue was purified by silica gel chromatography (hexane:ethyl acetate = 2:1) to give the titled compound 77 mg (yield 78%) as an oil.
¹H-NMR δ(CDCl₃, 270MHz) 0.83 and 1.20 (total 3H, t, J=6.9 Hz), 1.65-2.33 (7H, m), 3.08-3.18 (1H, m), 3.43 (1H, dt, J= 11, 2.3 Hz), 3.56 and 3.64 (total 1H, dt, J= 11, 2.5 Hz), 3.79-3.88 and 4.02-4.18 (total 2H, m), 3.98 and 4.21 (total 1H, br s), 7.32-7.76 (6H, m), 8.44 (1H, br s), 8.69 (1H, br s).

### (2) Syn-6-(5-phenyl-3-pyridyl)-2-azabicyclo[2. 2. 2]octane

Iodine 250 mg (0.98 mmol) and hexamethyldisilane 0.50 mL (2.5 mmol) were mixed and stirred at 100 °C for 10 min. After allowed to cool, to the mixture was added a solution of *syn*-2-ethoxycarbonyl-6-(5-phenyl-3-pyridyl)-2-azabicyclo[2. 2. 2]octane 70 mg (0.21 mmol) in 5 mL of 1,2-dichloroethane, and stirred at 50 °C for 2 hours. Methanol 1 mL was added to the mixture and the mixture was concentrated in vacuo. Aq. 1 N NaOH solution and aq. Na₂S₂O₃ solution were added thereto. The mixture was extracted with chloroform. The extracts were dried over anhydrous Na₂SO₄ and concentrated in vacuo and the residue was purified by silica gel chromatography (chloroform : methanol : conc. aq. ammonia solution = 100 : 10 : 1) to give the titled compound 40 mg (yield 73%) as an oil.
¹H-NMR δ(CDCl₃, 270MHz) 1.64-2.35 (7H, m), 3.17 (1H, dd, J= 11, 7.3 Hz), 3.22 (1H, br d, J= 11 Hz), 3.31 (1H, dt, J= 11, 2.6 Hz), 3.41 (1H, br s), 7.34-7.48 (3H, m), 7.51-7.58 (2H, m), 7.86 (1H, t, J= 2 Hz), 8.51 (1H, d, J= 2.0 Hz), 8.60 (1H, d, J= 2.3 Hz).

### Example 30

### Syn-5-(3-pyridyl)-2-azabicyclo[2. 2. 1]heptane

### (1) 2-Ethoxycarbonyl-2-azabicyclo[2. 2. 1]hept-4-ene

37% Formalin 80 g was diluted with water 50 mL and ammonium chloride 52.0 g (970 mmol) was added thereto and stirred at room temperature for 1.5 hours. To the mixture was added cyclopentadiene 51 g (770 mmol), stirred for 2 hours and left for 2 days. The reaction mixture was washed with ethyl acetate. The aqueous layer was made basic with KOH, and extracted with chloroform. The extracts were dried over anhydrous Na₂SO₄ and concentrated in vacuo. The residue was dissolved in methylene chloride 200 mL, and triethylamine 130 mL (920 mmol) was added thereto. The mixture was cooled in ice bath and ethyl chloroformate 73 mL (770 mmol) was added dropwise thereto. The mixture was warmed to room temperature over 1 hour. Water was added thereto and the organic layer was separated and the aqueous layer was extracted with chloroform. The organic layers were combined, dried over anhydrous Na₂SO₄ and concentrated in vacuo. The residue was distilled under reduced pressure and fractions of boiling point of 65-72 °C at 0.1 mmHg were collected to give the titled compound 11.0 g (yield 9%).
¹H-NMR δ(CDCl₃, 270Mhz) 1.20-1.35 (3H, m), 1.58 (2H, s), 2.57-2.72 (1H, m), 3.19 (1H, br s), 3.32-3.40 (1H, m), 4.03-4.22 (2H, m), 4.66 and 4.76 (total 1H, br s), 6.25-6.48 (2H, m).

### (2) 2-Ethoxycarbonyl-5-hydroxy-2-azabicyclo[2. 2. 1]heptane

A solution of 55 mL (55 mmol) of 1 M borane/THF was added at -78 °C to a solution of 2-ethoxycarbonyl-2-azabicyclo[2. 2. 1]hept-4-ene 10.0 g (55 mmol) in 50 mL of THF. The mixture was gradually warmed to 0 °C and stirred for 4 hours. Aq. 4 N NaOH solution 20 mL and aq. 30% hydrogen peroxide solution 20 mL were added thereto, and stirred at room temperature for 2 hours. Water was added thereto and the mixture was extracted with ethyl acetate. The extracts were washed with water and brine, dried over anhydrous MgSO₄ and concentrated in vacuo. The residue was purified by silica gel chromatography (hexane:ethyl acetate = 1:1) to give the titled compound 4.66 g (yield 41%) as an oil.
¹H-NMR δ(CDCl₃, 270MHz) 1.20-1.32 (3H, m), 1.38-1.92 (4H, m), 2.54 (1H, br s), 2.82 and 2.88 (1H, br d, J= 10Hz), 3.12-3.25 (1H, m), 3.94-4.20 (4H, m).

### (3) 2-Ethoxycarbonyl-2-azabicyclo[2. 2. 1]heptan-5-one

Dess-Martin's reagent 348 mg (0.82 mmol: J. Org. Chem., 48, 4155(1983)) was added to a solution of 2-ethoxycarbonyl-5-hydroxy-2-azabicyclo[2. 2. 1]heptane 135 mg (0.68 mmol) in 2 ml, of methylene chloride with cooling in an ice bath. The mixture was gradually warmed to room temperature and stirred for 4 hours. The reaction mixture was diluted with ether, and insoluble substances were filtered off. The filtrate was concentrated in vacuo and the residue was purified by silica gel chromatography (hexane:ethyl acetate = 1:1) to give the titled compound 96 mg (yield 71%) as an oil.
¹H-NMR δ(CDCl₃, 270MHz) 1.26 (3H, t, J= 7.2 Hz), 1.72 and 1.76 (total 1H, dd, J= 3.0, 1.7 Hz), 1.86-1.98 (1H, m), 1.99 and 2.05 (total 1H, d, J= 4.0 Hz), 2.22 and 2.28 (total 1H, dd, J= 4.6, 1.6 Hz), 2.84-2.91 (1H, m), 3.12-3.28 (1H, m), 3.51 (1H, ddd, J=9.6, 3.0, 2.0 Hz), 4.09-4.35 (2H, m), 4.15 (2H, q, J= 7.2 Hz).

### (4) 2-Ethoxycarbony1-5-hydroxy-5-(3-pyridyl)-2-azabicyclo[2. 2. 1]heptane

2.5 Milliliter(4 mmol) of a solution of 1.6 N n-butyl lithium in hexane was added dropwise at -100 °C to a solution of 3-bromopyridine 583 mg (3.7 mmol) in 10 mL of THF, and stirred for 5 min. A solution of 2-ethoxycarbonyl-2-azabicyclo[2. 2. 1]heptan-5-one 450 mg (2.46 mmol) in 5 mL of THF was added thereto and stirred for 15 min. After water was added and warmed to room temperature, the mixture was extracted with ethyl acetate. The extracts were washed with brine, dried over anhydrous Na₂SO₄, concentrated in vacuo and the residue was purified by silica gel chromatography (chloroform:methanol = 30:1) to give the titled compound 375 mg (yield 61%) as an oil.
¹H-NMR δ(CDCl₃, 270MHz) 1.29 (3H, t, J=7.3 Hz), 1.65-1.80 (1H, m), 1.71 (2H,s), 1.84 (1H, s, -OH), 2.35-2.52 (1H, m), 2.67 (1H, br s), 3.27 (1H, d, J= 9.6 Hz), 3.47 (1H, br d, J=9.6 Hz), 4.19 (2H, q, J=7.3 Hz), 4.46 (1H, br s), 7.30 (1H, ddd, J= 7.9, 4.6, 0.7 Hz), 7.83 (1H, ddd, J= 7.6, 2.3, 0.8 Hz), 8.52 (1H, dd, J= 4.6, 1.7 Hz), 8.73 (1H, d, J= 2.0 Hz).

### (5) Syn-2-ethoxycarbonyl-5-(3-pyridyl)-2-azabicyclo[2. 2. 1]heptane

One mL of thionyl chloride was added to a solution of 2-ethoxycarbonyl-5-hydroxy-5-(3-pyridyl)-2-azabicyclo[2. 2. 1]heptane 300 mg (1.15 mmol) in methylene chloride (2 mL) with cooling in an ice bath. The mixture was warmed to room temperature, stirred for 2 hours, concentrated, and aq. 1 N NaOH solution was added to the residue. The mixture was extracted with chloroform and dried over anhydrous Na₂SO₄. After activated carbon was added and stirred, the mixture was filtered. The filtrate was concentrated under reduced pressure and diluted with ethyl acetate 10mL. 10% Palladium/carbon 120 mg was added thereto, and the mixture was stirred under a hydrogen atmosphere for 1 hour. After the catalyst was filtered off, the filtrate was concentrated in vacuo and the residue was purified by silica gel chromatography (chloroform:methanol = 40:1 → 20:1) to give the titled compound 74 mg (yield 26%) as an oil.
¹H-NMR δ(CDCl₃, 270MHz) 0.78 and 1.16 (total 3H, t, J= 6.9Hz), 1.57 (1H, ddd, J= 13, 5.3, 2.3 Hz), 1.73-1.90 (2H, m), 2.12-2.32 (1H, m), 2.71 (1H, br s), 3.14 and 3.23 (1H, d, J=10 Hz), 3.30-3.52 (2H, m), 3.53-3.75 and 3.88-4.05 (total 2H, m), 4.24 and 4.26 (total 1H, br s), 7.15-7.25 (1H, m), 7.41 and 7.54 (total 1H, br d, J= 8 Hz), 8.40-8.42 (2H, m).

### (6) Syn-5-(3-pyridyl)-2-azabicyclo[2. 2. 1]heptane

*Syn*-2-ethoxycarbonyl-5-(3-pyridyl)-2-azabicyclo[2. 2. 1]heptane 70 mg (0.3 mmol) was subjected to removal of the ethoxycarbonyl group by the same method as that in Example 29(2) to give the titled compound 30 mg (yield 62%) as an oil.
¹H-NMR δ(CDCl₃, 270MHz) 1.54 (1H, ddd, J= 13, 5.3, 2.3 Hz), 1.71-1.79 (1H, m), 1.83 (1H, ddd, J= 9.6, 4.0, 2.0 Hz), 2.11 (1H, dddd, J= 12, 12, 4.6, 3.3 Hz), 2.53 (1H, br s), 2.71 (1H, dd, J= 9.6, 1.0 Hz), 2.99 (1H, dt, J=9.6, 3.3 Hz), 3.30 (1H, ddd, J= 12, 5.3, 2.6 Hz), 3.45 (1H, br s), 7.27 (1H, ddd, J= 7.9, 4.0, 0.7 Hz), 7.54 (1H, br d, J=7.9 Hz), 8.48 (1H, dd, J= 5.0, 1.7 Hz), 8.50 (1H, d, 3.3 Hz).

### Example 31

### Syn-8-(3-pyridyl)-7-azabicyclo[3. 2. 2.]nonane

### (1) 7-Ethoxycarbonyl-7-azabicyclo[3. 2. 2]non-8-ene

Boron trifluoride diethyl ether complex 5.0 g was added to a solution of methylene bisurethane 24.0 g (126 mmol) in 200 mL of toluene, and the mixture was warmed to 80 °C. 1,3-Cycloheptadiene 14.7 mL (156 mmol) was added dropwise thereto and stirred at the same temperature for 6 hours. The mixture was allowed to cool, washed with sat. aq. NaHO₃, water and brine, and dried over anhydrous Na₂SO₄. The residue was distilled under reduced pressure and fractions of boiling point of 115-125 °C at 1 mmHg were collected to give the titled compound 4.27 g (yield 17%).
¹H-NMR δ(CDCl₃, 270MHz) 1.26 (3H, t, J= 7.3 Hz), 1.40-1.70 (3H, m), 1.72-1.90 (1H, m), 2.50-2.64 91H, m), 3.15-3.24 (1H, m), 3.47 and 3.54 (1H, d, J= 11 Hz), 4.08-4.20 (2H, m), 4.52-4.60 and 4.65-4.73 (total 1H, m), 6.00-6.27 (2H, m).

### (2) 7-Ethoxycarbonyl-7-azabicyclo[3. 2. 2]nonan-8-one

Phenylselenenyl chloride 4.95 g (5.94 mmol) was added at -78 °C to a solution of 7-ethoxycarbonyl-7-azabicyclo[3. 2. 2]non-8-ene 4.2 g (22 mmol) in methylene chloride (100 mL), and the mixture was gradually warmed to room temperature and stirred overnight. The mixture was concentrated in vacuo and the residue was purified by silica gel chromatography (hexane:ethyl acetate = 5:1) to give the adduct 8.15 g as an oil. DBU 10 mL was added to the adduct, and stirred at 130 °C for 2 hours. The mixture was allowed to cool, poured into water, and extracted with ether. The extracts were washed with 1 N HCl and brine, dried over anhydrous MgSO₄, and concentrated in vacuo. 20% HCl 100 mL and 1,4-dioxane 100 mL was added to the residue and heated under reflux for 2 hours. The reaction mixture was extracted with chloroform. The extracts were washed with brine, dried over anhydrous MgSO₄, concentrated under reduced pressure, and the residue was purified by silica gel chromatography (hexane:ethyl acetate = 2:1) to give the titled compound 1.12 g (yield 25%) as an oil.
¹H-NMRδ (CDCl₃, 270MHz) 1.26 and 1.29 (total 3H, t, J=7.2 Hz), 1.40-1.95 (6H, m), 2.41 (2H, br s), 2.45-2.60 (1H, m), 3.47-3.62 (2H, m), 4. 10-4.22 (2H, m), 4.59 and 4.73 (total 1H, t, J= 4.3 Hz).

### (3) 7-Ethoxycarbonyl-8-hydroxy-8-(3-pyridyl)-7-azabicyclo[3. 2. 2]nonane

The titled compound 431 mg (yield 63%) was obtained as an oil from 7-ethoxycarbonyl-7-azabicyclo[3. 2. 2]nonan-8-one 500 mg (2.4 mmol) by the same method as that in Example 30(4).
¹H-NMR δ(CDCl₃, 270MHz) 1.04 and 1.18 (total 3H, J= 6.9 Hz), 1.53-2.00 (5H, m), 2.09 (1H, t like, J= 16 Hz), 2.23-2.50 (2H, m), 2.56 (1H, dt, J= 6.3, 14 Hz), 3.17 (1H, dt, J= 13, 3.6), 3.47 (1H, tt, J= 13, 2.3 Hz), 3.92-4.10 (2H, m), 4.35 and 4.51 (total 1H, t, J= 4.0Hz), 7.19-7.28 (1H, m), 7.73 and 7.81 (total 1H, ddd, J= 8.3, 2.6, 1.7 Hz), 8.45 and 8.46 (total 1H, t, J= 1.7 Hz), 8.70 and 8.78 (total 1H, d, J= 2.6 Hz).

### (4) 7-Ethoxycarbonyl-8-(3-pyridyl)-7-azabicyclo[3. 2. 2]nonane

The titled compound 100 mg (yield 53%) was obtained as an oil from 7-ethoxycarbonyl-8-hydroxy-8-(3-pyridyl)-7-azabicyclo[3. 2. 2]nonane 200 mg (0.69 mmol) by the same method as that in Example 30(5).
¹H-NMR δ(CDCl₃, 270MHz) 1.05-1.35 (total 3H, m), 1.45-2.50 (9H, m), 3.13-3.75 (3H, m), 4.00-4.52 (3H, m), 7.17-7.33 (1H, m), 7.43-7.50 and 7.63-7.70 (total 1H, m), 8.40-8.52 and 8.60-8.65 (total 2H, m).

### (5) Syn-8-(3-pyridyl)-7-azabicyclo[3. 2. 2]nonane

The titled compound, less polar isomer, 37 mg (yield 30%) was obtained as an oil from 7-ethoxycarbonyl-8-(3-pyridyl)-7-azabicyclo[3. 2. 2]nonane 95 mg (0.35 mmol) by the same method as that in Example 30(6).
¹H-NMR δ(CDCl₃, 270MHz) 1.65-2.25 (9H, m), 2.99-3.15 (3H, m), 3.24 (1H, ddd, J= 11.5, 5.0, 1.3 Hz), 7.25 (1H, dd, J= 8.9, 5.0 Hz), 7.77 (1H, dt, J= 7.9, 1.7 Hz), 8.45 (1H, dd, J= 5.0, 1.7 Hz), 8.55 (1H, d, J=2.3 Hz).

The structures of the compounds obtained in Example 1 to 31 are as follows:

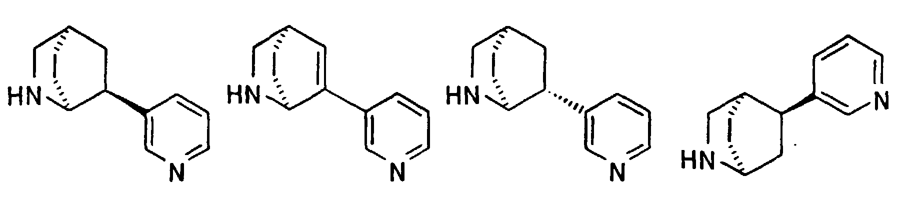

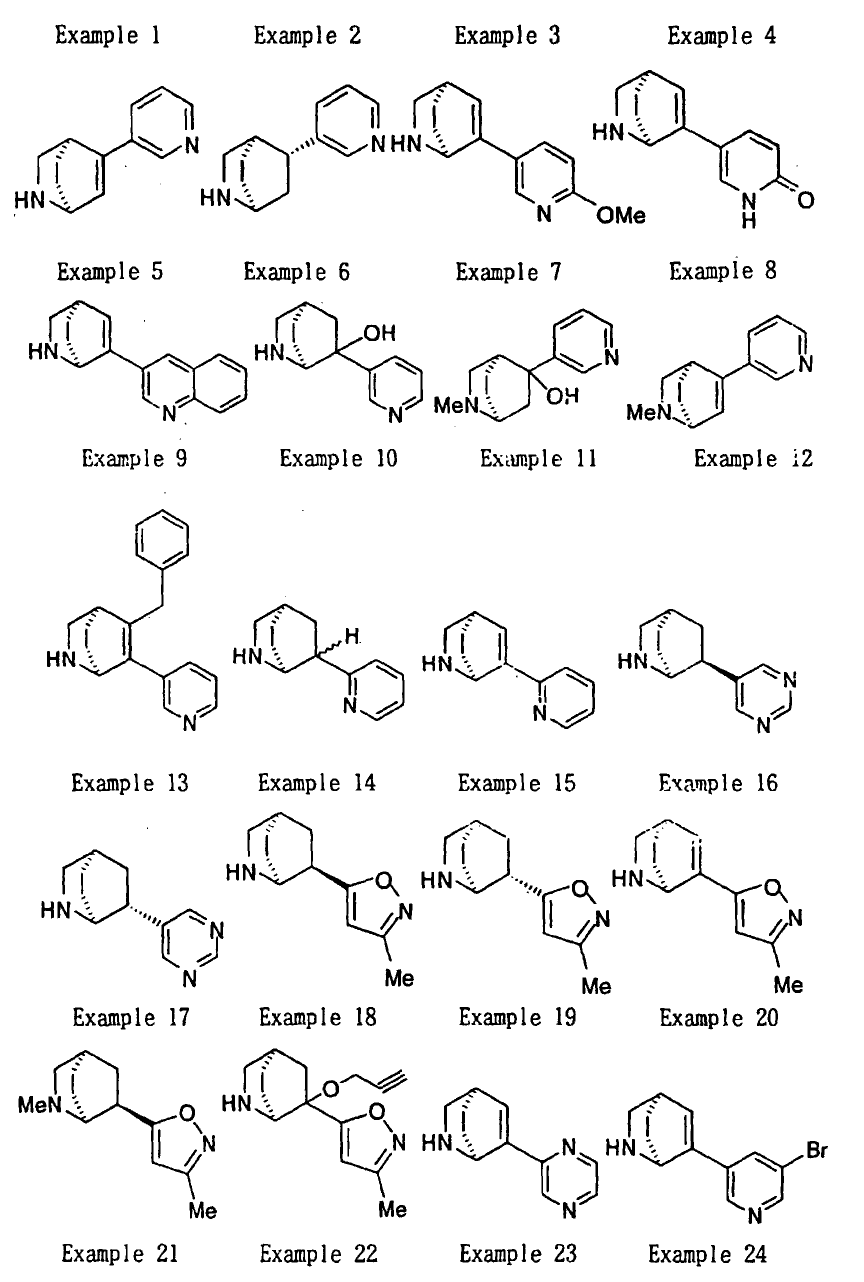

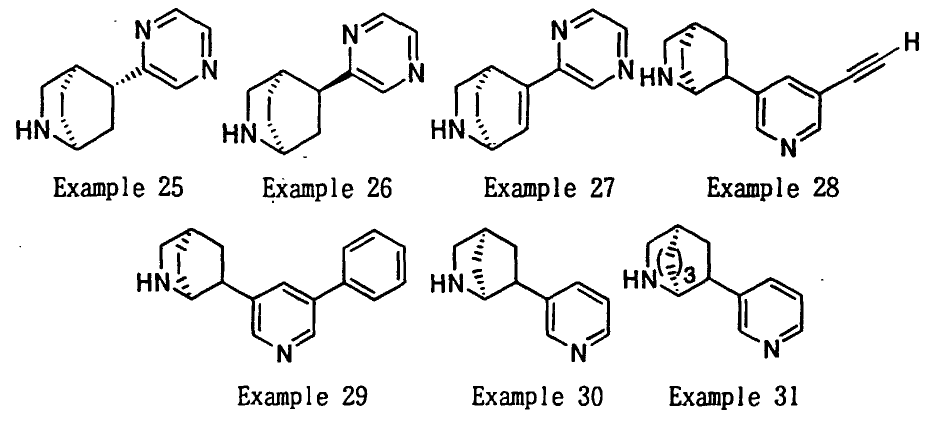

### Example 32

### Nicotinic acetylcholine receptor binding assay

### (Preparation of a membrane fraction)

The membrane fraction was prepared in accordance with the protocol described in Brain Res., 100, 81-87 (1977). Thus, the rat was decapitated, and the whole brain was removed and homogenized with 15 volumes of cold 0.32 M aqueous sucrose solution using a Teflon homogenizer. The homogenate was centrifuged at 4°C and 1,000 x g for 10 mm. and the supernatant obtained was centrifuged at 4°C and 20,000 x g for 20 min. Then, ice-cold distilled water was added to the pellet, and the pellet was suspended using Hiscotron®. The suspension was centrifuged at 4°C and 8,000 x g for 20 min. and the supernatant and the buffy coat layer were recovered and centrifuged again at 4°C and 40,000 x g for 20 min. The pellet obtained was suspended in cold distilled water and stored at -80°C until used.

### (Receptor binding experiment)

The receptor binding experiment was performed in accordance with the protocol described in J. Pharamcol. Exp. Ther., 270, 310-318 (1994). Thus, the rat whole brain membrane fraction (ca 200 µg) was incubated in BSS-Tris buffer [composition mM: NaCl 120, KCl 5, CaCl₂2, MgCl₂2, Tris-HCl 50 (pH=7.4, at 4°C)] containing 1 nM of ³H-cytidine and 10 nM of the test drug at 4°C for 75 min. To stop the reaction, 4 ml of cold BSS-Tris buffer was added and the reaction mixture was immediately suction-filtered on GF/B glass fiber filter paper which had been pre-soaked in 0.5% polyethyleneimine. The filter paper was recovered, 4 ml of ACS-II (Amersham, TM) was added, and the ³H radioactivity was determined with a scintillation counter. Based on the value (specific binding) obtained after subtraction of the ³H-binding (nonspecific binding) amount in the presence of 10 µM (-)nicotine, the inhibition rate of ³H-cytidine binding at the 10 nM concentration of each drug was calculated. The data are shown in Table 1.

**Table 1**

| Compound | Inhibition of ³H-Cytidine binding |
|---|---|
| Example 1 | 94 % |
| Example 16 | 93 % |
| Example 18 | 84 % |
| Example 23 | 39 % |
| Example 24 | 92 % |
| Example 28 | 91 % |
| Example 29 | 86 % |
| Example 30 | 87 % |
| Example 31 | 84 % |

### Industrial Applicability

The present invention can provide a nicotinic acetylcholine receptor agonist useful as a medicament for treating a neurodegenerative disorder such as Alzheimer's disorder, Parkinson's disease and the like, and as an analgesic.

## Claims

1. A nicotinic acetylcholine receptor agonist comprising a 2-azabicyclo compound of formula: 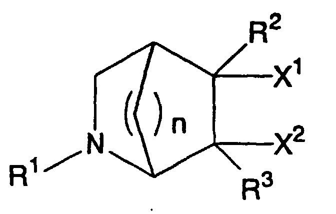
wherein R¹ is hydrogen or alkyl;
one of R² and R³ is optionally substituted nitrogen-containing heteroaryl; the other of R² and R³ is hydrogen, alkyl, alkenyl, alkynyl or aralkyl:
n is 1, 2 or 3;
one of X¹ and X² is hydrogen, and the other of X¹ and X² is hydrogen, hydroxy, halogen, alkoxy, alkenyloxy or alkynyloxy, or X¹ and X² are taken together with the solid line to form double bond;
or a pharmaceutically acceptable salt thereof.

2. A nicotinic acetylcholine receptor agonist according to Claim 1 wherein one of R² and R³ is optionally substituted pyridyl, optionally substituted pyrazinyl, optionally substituted pyrimidinyl, optionally substituted pyridazinyl, optionally substituted quinolyl, optionally substituted isoxazolyl, optionally substituted isothiazolyl or optionally substituted pyridonyl.

3. A nicotinic acetylcholine receptor agonist according to Claim 2 wherein one of R² and R³ is 3-pyridyl, 5-pyrimidinyl, 2-pyrazinyl, 3-methyl-5-isoxazolyl, 5-ethynyl-3-pyridyl or 5-phenyl-3-pyridyl.

4. A nicotinic acetylcholine receptor agonist according to any one of Claim 1 to Claim 3 wherein the other of R² and R³ is hydrogen.

5. A nicotinic acetylcholine receptor agonist according to any one of Claim 1 to Claim 4 wherein n is 2.

6. A nicotinic acetylcholine receptor agonist according to any one of Claim 1 to Claim 5 wherein both of X¹ and X² are hydrogen atoms or X¹ and X² are taken together with the solid line to form double bond.

7. A nicotinic acetylcholine receptor agonist according to any one of Claim 1 to Claim 6 wherein R¹ is hydrogen.

8. A method for stimulating nicotinic acetylcholine receptors comprising administrating to a patient in need thereof a 2-azabicyclo compound of the formula: 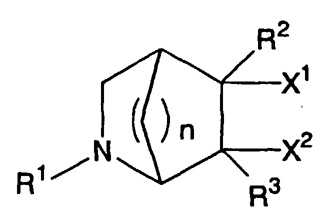 wherein R¹, R², R³, n, X¹ and X² are as defined in Claim 1; or a pharmaceutically acceptable salt thereof.

9. Use of a 2-azabicyclo compound of the formula: 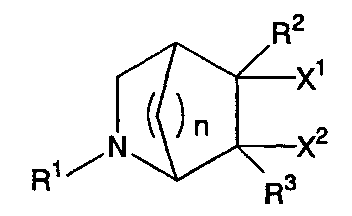 wherein R¹, R², R³, n, X¹ and X² are as defined in Claim 1; or a pharmaceutically acceptable salt thereof for the manufacture of a nicotinic acetylcholine receptor agonist.

10. A 2-azabicyclo compound of the formula: 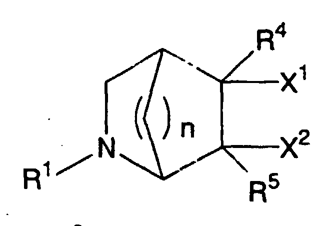
wherein R¹, n, X¹ and X² are as defined in Claim 1:
one of R⁴ and R⁵ is optionally substituted 3-pyridyl, optionally substituted 5-pyrimidinyl, optionally substituted 2-pyrazinyl, optionally substituted 5-isoxazolyl or optionally substituted pyridonyl, provided that X¹ and X² are taken together with the solid line to form double bond in case that one of R⁴ and R⁵ is optionally substituted 2-pyrazinyl;
the other of R⁴ and R⁵ is hydrogen, alkyl, alkenyl, alkynyl or aralkyl;
or a pharmaceutically acceptable salt thereof.

11. A 2-azabicyclo compound or a pharmaceutically acceptable salt thereof according to Claim 10 wherein one of R⁴ and R⁵ is 3-pyridyl, 5-pyrimidinyl, 2-pyrazinyl, 3-methyl-5-isoxazolyl, 5-ethynyl-3-pyridyl or 5-phenyl-3-pyridyl.

12. A 2-azabicyclo compound or a pharmaceutically acceptable salt thereof according to Claim 10 or Claim 11 wherein the other of R⁴ and R⁵ is hydrogen.

13. A 2-azabicyclo compound or a pharmaceutically acceptable salt thereof according to any one of Claim 10 to Claim 12 wherein n is 2.

14. A 2-azabicyclo compound or a pharmaceutically acceptable salt thereof according to any one of Claim 10 to Claim 13 wherein both of X¹ and X² are hydrogen atoms or X¹ and X² are taken together with the solid line to form double bond.

15. A 2-azabicyclo compound or a pharmaceutically acceptable salt thereof according to any one of Claim 10 to Claim 14 wherein R¹ is hydrogen.

16. A medicament comprising a 2-azabicyclo compound or a pharmaceutically acceptable salt thereof according to any one of Claim 10 to Claim 15.
